# EUROPEAN PATENT APPLICATION

(11) **EP 2 426 117 A1**
(43) Date of publication of application: **07.03.2012**
(21) Application number: 10769858.1
(22) Date of filing: 28.04.2010
(51) Int. Cl.: C07D 333/36, A61K 31/381, A61K 31/4025, A61K 31/4535, A61P 3/04, A61P 3/06, A61P 3/10, A61P 5/26

(54) **THIOPHENE DERIVATIVE**

(30) Priority: 30.04.2009 JP 2009110804
(71) Applicant: Sumitomo Chemical Company, Limited, Tokyo 104-8260 (JP)
(72) Inventor: HARADA, Koichiro, Toyonaka-shi Osaka 561-0852 (JP); MOTONAGA, Kozo, Ibaraki-shi Osaka 567-0841 (JP); SAITO, Koichi, Osaka-shi Osaka 530-0005 (JP); TANAKA, Akio, Kobe-shi Hyogo 658-0073 (JP)
(74) Representative: Duckworth, Timothy John
(86) International application number: PCT/JP2010/057917
(87) International publication number: WO 2010/126167

(57) **Abstract**

The present invention provides a compound represented by the formula (I) or its salt, solvate, or physiologically functional derivative; and a pharmaceutical composition which is useful for treatment or prevention of conditions or disorders having sensitivity to selective androgen receptor modulation, the composition comprising the above-described compound; among others:

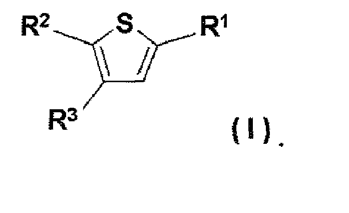

## Description

### TECHNICAL FIELD

The present invention relates to a thiophene derivative, more particularly, a novel thiophene derivative having an ability of modulating androgen receptor activity manifestation, and its pharmaceutically acceptable salt, and use thereof.

### BACKGROUND ART

It has been clarified that a substance having an ability of modulating androgen receptor (hereinafter, referred to as AR in some cases) activity manifestation (hereinafter, referred to as AR modulator in some cases) exerts a remarkable influence on the control of neprilysin (hereinafter, referred to as NEP in some cases) activity manifestation, and that the AR activity manifestation-modulating ability of the substance is closely correlated with the NEP activity manifestation-controlling ability, specifically, that the AR modulator controls NEP gene manifestation and NEP enzyme activity in neurocytes, further, controls the extracellular secretion amount of an amyloid β protein (hereinafter, referred to as Aβ in some cases) in neurocytes (International Publication WO 05/073395).

Conventional AR modulators function as an AR agonist, an AR partial agonist, an AR antagonist or an AR partial antagonist. For example, AR antagonists such as cyproterone, flutamide and casodex are used as a therapeutic medicine for benign prostatic hypertrophy and prostatic cancer. In contrast, AR agonists are used as a therapeutic medicine for male hypogonadism, wasting disease, osteoporosis and ADAM and the like. Specifically, testosterone propionate and methyltestosterone are used for an androgen replacement therapy, and effectiveness thereof on improvement of the above-described diseases and conditions is recognized. The above-described steroid preparations, however, have side effects such as hepatic function disorder and gastrointestinal disorder, and additionally, act excessively on prostate in the case of administration to male patients, particularly, elder patients, thus, there is a possibility of promotion of onset and symptomatic worsening of androgen-dependent tumors and prostatic hypertrophy.

Therefore, there is a desire for a therapeutic medicine composed of a non-steroidal compound as a selective androgen receptor modulator (hereinafter, referred to as SARM in some cases) having no potential side effects observed in conventional androgen steroid preparations, in treatment and prevention of androgen receptor-mediated diseases. Theoretically, SARM is required to manifest smaller side effects, particularly, to cause no damage of prostate, and to have all beneficial effects of endogenous androgen, in treatment or prevention against conditions or disorders having sensitivity to selective androgen receptor modulation.

### DISCLOSURE OF THE INVENTION

The present invention has an object of providing a compound having an androgen receptor activity manifestation-modulating ability and its pharmaceutically acceptable salt, a pharmaceutical composition which is useful for treatment or prevention of conditions or disorders having sensitivity to selective androgen receptor modulation including amyloid β protein-associated diseases, and the composition comprising the above-described compound; among others.

The present invention provides a specific thiophene derivative having an androgen receptor activity manifestation-modulating ability and which is useful for treatment or prevention of conditions or disorders having sensitivity to selective androgen receptor modulation. Among SARM substances, substances showing an AR agonist activity in neurocytes suppress the extracellular secretion amount of Aβ, thus, the thiophene derivative having an androgen receptor activity manifestation-modulating ability can be a pharmaceutical composition for amyloid β protein-associated diseases.

That is, the present provides:
[1] A compound represented by the formula (T): 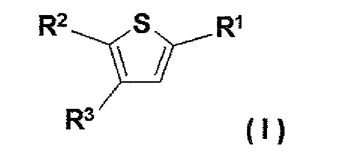 [wherein:
   R¹ is a group represented by the formula (II): 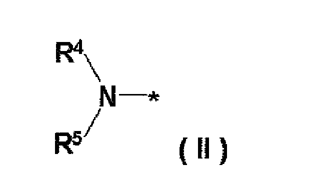
   {wherein:
   R⁴ represents -(Q¹)x-R⁶;
   Q¹ represents alkylene;
   X is 0 or 1;
   R⁶ represents hydrogen, alkyl, cycloalkyl, alkenyl, alkynyl, halogen, haloalkyl or alkoxy;
   R⁵ represents -(Q²)y¹-(Q³)y²-R⁷;
   Q² represents alkylene;
   Q³ represents -C(O)- or C (s)-;
   y¹ is 0 or 1;
   y² is 0 or 1;
   R⁷ represents hydrogen, alkyl, cycloalkyl, alkenyl, alkynyl, halogen, haloalkyl, hydroxy, alkoxy, cyano, - N(R⁸) (R⁹) or -CH(R¹⁰)(R¹¹);
   R⁸ and R⁹ represent each independently hydrogen, alkyl, cycloalkyl, alkenyl or alkynyl;
   R¹⁰ and R¹¹ represent each independently alkyl, cycloalkyl, alkenyl, alkynyl, halogen, haloalkyl, hydroxy, alkoxy or cyano, alternatively R¹⁰ and R¹¹ together form alkylenedioxy (if either R¹⁰ or R¹¹ is hydroxy, another is a moiety other than hydroxy and alkoxy)}
   or a group represented by the formula (III): 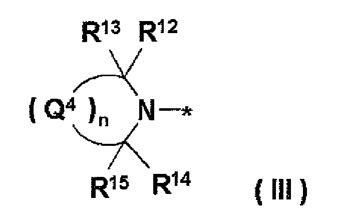 {wherein:
   Q⁴ represents optionally substituted methylene;
   n is 1 to 5;
   R¹² and R¹³ represent each independently -(Q⁵)z-R¹⁶, alternatively form = O or = S together with a carbon atom to which they are bonded;
   Q⁵ represents alkylene;
   z is 0 or 1;
   R¹⁶ represents hydrogen, alkyl, cycloalkyl, alkenyl, alkynyl, halogen, haloalkyl, hydroxy, alkoxy, cyano, formyl, - CH=N-R¹⁷, -N(R¹⁸) (R¹⁹) or -CH(R²⁰) (R²¹);
   R¹⁷ represents hydrogen, alkyl, cycloalkyl, alkenyl, alkynyl or hydroxy;
   R¹⁸ and R¹⁹ represent each independently hydrogen, alkyl, cycloalkyl, alkenyl or alkynyl;
   R²⁰ and R²¹ represent each independently alkyl, cycloalkyl, alkenyl, alkynyl, halogen, haloalkyl, hydroxy, alkoxy or cyano (if either R²⁰ or R²¹ is hydroxy, another is a moiety other than hydroxy and alkoxy);
   R¹⁴ and R¹⁵ represent each independently -(Q⁶)w-R²²;
   Q⁶ represents alkylene;
   w is 0 or 1;
   R²² represents hydrogen, alkyl, cycloalkyl, alkenyl, alkynyl, halogen, haloalkyl, hydroxy, alkoxy, cyano, formyl, - CH=N-R²³, -N(R²⁴)(R²⁵) or -CH(R²⁶)(R²⁷);
   R²³ represents hydrogen, alkyl, cycloalkyl, alkenyl, alkynyl or hydroxy;
   R²⁴ and R²⁵ represent each independently hydrogen, alkyl, cycloalkyl, alkenyl or alkynyl;
   R²⁶ and R²⁷ represent each independently alkyl, cycloalkyl, alkenyl, alkynyl, halogen, haloalkyl, hydroxy, alkoxy or cyano (if either R²⁶ or R²⁷ is hydroxy, another is a moiety other than hydroxy and alkoxy)};
   R² represents cyano, formyl, nitro, halogen or haloalkyl;
   R³ represents cyano, nitro, halogen, haloalkyl, alkyl, alkenyl, alkynyl or alkoxy]
   or its salt, solvate, or physiologically functional derivative:
   excluding the following compounds:
      3-methyl-2-nitro-5-(piperidin-1-yl)thiophene;
      3-ethyl-2-nitro-5-(piperidin-1-yl)thiophene;
      2-nitro-5-(piperidin-1-yl)-3-(n-propyl)thiophene;
      2-nitro-5-(piperidin-1-yl)-3-(i-propyl)thiophene;
      3- (t-butyl)-2-nitro-5-(piperidin-1-yl)thiophene; and
      3-(n-hexyl)-2-nitro-5-(piperidin-1-yl)thiophene;
[2] The compound according to [1], wherein the alkyl is a C1-C6 alkyl, the alkenyl is a C2-C6 alkenyl, the alkynyl is a C2-C6 alkynyl, the haloalkyl is a C1-C6 haloalkyl, the cycloalkyl is a C3-C6 cycloalkyl, the alkylene is a C1-C6 alkylene and the alkoxy is a C1-C6 alkoxy;
[3] The compound according to [2], wherein the alkylene is a C1-C2 alkylene, the haloalkyl is trifluoromethyl and the cycloalkyl is cyclopropyl;
[4] The compound according to any one of [1] to [3], wherein R² represents cyano, formyl, halogen or haloalkyl;
[5] The compound according to [4], wherein R² is cyano;
[6] The compound according to any one of [1] to [5], wherein R³ is cyano, halogen or haloalkyl;
[7] The compound according to [6], wherein R³ is a halogen or haloalkyl;
[8] The compound according to [7], wherein R³ is bromo or trifluoromethyl;
[9] The compound according to any one of [1] to [8], wherein Q¹ is methylene or ethylene;
[10] The compound according to any one of [1] to [9], wherein R⁶ is hydrogen, alkyl, cycloalkyl, alkenyl, haloalkyl or alkoxy;
[11] The compound according to any one of [1] to [10], wherein R⁶ is cyclopropyl, vinyl, trifluoromethyl or methoxy;
[12] The compound according to any one of [1] to [11], wherein Q² is methylene or ethylene;
[13] The compound according to any one of [1] to [12], wherein Q³ is -C(O)- or -C(S)-;
[14] The compound according to any one of [1] to [13], wherein R⁷ is hydrogen, alkyl, alkenyl, hydroxy, alkoxy, cyano, -N(R⁸)(R⁹) or -CH(R¹⁰)(R¹¹) in which R⁸ and R⁹ represent each independently hydrogen or alkyl, R¹⁰ and R¹¹ represent each independently alkyl, haloalkyl or hydroxy, alternatively R¹⁰ and R¹¹ together form alkylenedioxy;
[15] The compound according to any one of [1] to [14], wherein Q⁴ is methylene optionally substituted with a substituent selected from the group consisting of a halogen, hydroxy, C1-C6 alkyl, C2-C6 alkenyl, C2-C6 alkynyl, C1-C6 haloalkyl, C3-C6 cycloalkyl and C1-C6 alkoxy;
[16] The compound according to any one of [1] to [15], wherein Q⁵ is methylene;
[17] The compound according to any one of [1] to [16], wherein R¹⁶ is hydrogen, cycloalkyl, alkenyl, halogen, haloalkyl, hydroxy, formyl, -CH=N-R¹⁷ or -CH(R²⁰)(R²¹) in which R¹⁷ represents hydroxy and R²⁰ and R²¹ represent each independently alkyl, haloalkyl or hydroxy (excluding a case in which R²⁰ and R²¹ are simultaneously hydroxy);
[18] The compound according to [17], wherein either R²⁰ or R²¹ is a haloalkyl and another is hydroxy;
[19] The compound according to [18], wherein R²⁰ or R²¹ is trifluoromethyl;
[20] A compound selected from among
   3-bromo-5-[(cyclopropylmethyl)(propyl)amino]thiophene-2-carbaldehyde;
   3-bromo-5-[(cyclopropylmethyl)(propyl)amino]thiophene-2-carbonitrile;
   3-chloro-5-[(cyclopropylmethyl)(propyl)amino]thiophene-2-carbonitrile;
   5-[(cyclopropylmethyl)(propyl)amino]-2-(trifluoromethyl)thiophene-2-carbaldehyde;
   5-[(cyclopropylmethyl)(propyl)amino]-3-(trifluoromethyl)thiophene-2-carbonitrile;
   2-bromo-5-[(cyclopropylmethyl)(propyl)amino]thiophene-3-carbonitrile;
   5-[(cyclopropylmethyl)(propyl)amino]-2-(trifluoromethyl)thiophene-3-carbonitrile;
   3-bromo-5-(pyrrolidin-1-yl)thiophene-2-carbonitrile;
   3-bromo-5-(2-methylpiperidin-1-yl)thiophene-2-carbonitrile;
   3-bromo-5-(piperidin-1-yl)thiophene-2-carbonitrile;
   3-bromo-5-(3,5-dimethylpiperidin-1-yl)thiophene-2-carbonitrile;
   3-bromo-5-(4-methylpiperidin-1-yl)thiophene-2-carbonitrile;
   3-bromo-5-[(methyl)(2-methylpropyl)amino]thiophene-2-carbonitrile;
   3-bromo-5- (dipropylamino)thiophene-2-carbonitrile;
   3-bromo-5- (diallylamino)thiophene-2-carbonitrile;
   5-[bis(2-methoxyethyl)amino]-3-bromothiophene-2-carbonitrile;
   3-bromo-5-(2-ethylpiperidin-1-yl)thiophene-2-carbonitrile;
   3-bromo-5-(2-propylpiperidin-1-yl)thiophene-2-carbonitrile;
   N-(4-bromo-5-cyanothiophen-2-yl)-N-propylacetamide;
   N-(4-bromo-5-cyanothiophen-2-yl)-N-propylpropanamide;
   N-(4-bromo-5-cyanothiophen-2-yl)-N-propylbutanamide;
   N-(4-bromo-5-cyanothiophen-2-yl)-2-methyl-N-propylpropanamide;
   3-bromo-5-[(propyl)(2,2,2-trifluoroethyl)amino]thiophene-2-carbonitrile;
   N-(4-bromo-5-cyanothiophen-2-yl)-N-propylglycine ethyl ester;
   N2-(4-bromo-5-cyanothiophen-2-yl)-N-methyl-N2-propylglycinamide;
   N-(4-bromo-5-cyanothiophen-2-yl)-N-propylglycine;
   N2-(4-bromo-5-cyanothiophen-2-yl)-N2-propylglycinamide;
   3-bromo-5-[(cyanomethyl)(propyl)amino]thiophene-2-carbonitrile;
   N2-(4-bromo-5-cyanothiophen-2-yl)-N,N-dimethyl-N2-propylglycinamide;
   3-bromo-5-[(1,3-dioxolan-2-ylmethyl)amino]thiophene-2-carbonitrile;
   3-bromo-5-[(1,3-dioxolan-2-ylmethyl)(pxopyl)amino]thiophene-2-carbonitrile;
   3-bromo-5-[(2-oxoethyl)(propyl)amino]thiophene-2-carbonitrile;
   3-bromo-5-[propyl(3,3,3-trifluoro-2-hydroxypropyl)amino]thiophene-2-carbonitrile;
   3-bromo-5-[(2-hydroxypropyl)(propyl)amino]thiophene-2-carbonitrile;
   3-bromo-5-(2-ethyl-6-oxopiperidin-1-yl)thiophene-2-carbonitrile;
   3-bromo-5-(2-ethyl-5-thioxopiperidin-1-yl)thiophene-2-carbonitrile;
   N-(4-bromo-5-cyanothiophen-2-yl)-N-propylpropanthioamide;
   3-bromo-5-[2-(hydroxymethyl)piperidin-1-yl]thiophene-2-carbonitrile;
   3-bromo-5-(2-formylpiperidin-1-yl)thiophene-2-carbonitrile;
   3-bromo-5-[2-(2,2,2-trifluoro-1-hydroxyethyl)piperidin-1-yl]thiophene-2-carbonitrile;
   3-bromo-5-[2-(1-hydroxyethyl)piperidin-1-yl]thiophene-2-carbonitrile;
   3-bromo-5-{[2-(hydroxyimino)ethyl](propyl)amino}thiophene-2-carbonitrile;
   5-[2-(2,2,2-trifluoro-1-hydroxyethyl)piperidin-1-yl]-3-(trifluoromethyl)thiophene-2-carbonitrile;
   5-[(propyl)(2,2,2-trifluoroethyl)amino]-3-trifluoromethylthiophene-2-carbonitrile;
   5-[(propyl)(2,2,2-trifluoroethyl)amino]-3-methylthiophene-2-carbonitrile or
   5-(2-cyanopiperidin-1-yl)-3-bxomothiophene-2-carbonitrile,
   or its salt, solvate or physiologically functional derivative;
[21] The compound according to any one of [1] to [20], for use as an active therapeutic substance;
[22] The compound according to any one of [1] to [20], for use in treatment or prevention of conditions or disorders having sensitivity to selective androgen receptor modulation;
[23] The compound according to [22], wherein the condition or disorder having sensitivity to selective androgen receptor modulation is an androgen-dependent disease, proliferative disease or amyloidosis;
[24] The compound according to [22], wherein the condition or disorder having sensitivity to selective androgen receptor modulation is a malignant tumor containing an androgen receptor of breast, brain, skin, ovary, bladder, lymph node, liver, kidney, uterus, spleen, endometrium, lung, colon or prostate; prostatic hypertrophy, ADAM, trichosis, acne, seborrhea, androgenic alopecia, excessive hair growth, libido regression, sexual dysfunction, muscular weakness, aorta smooth muscle cell proliferation, Alzheimer's disease, mad cow disease, new variant Creutzfeldt-Jacob disease, familial amyloid polyneuropathy, osteoporosis, dyslipidemia, obesity, diabetes mellitus, depression, urine incontinence, arterial sclerosis, uterine fibroid disease or endometriosis;
[25] A pharmaceutical composition comprising the compound according to any one of [1] to [20] and a pharmaceutically acceptable carrier;
[26] Use of the compound according to any one of [1] to [20] in production of a medicine for use in treatment or prevention of conditions or disorders having sensitivity to selective androgen receptor modulation;
[27] The use according to [26], wherein the condition or disorder having sensitivity to selective androgen receptor modulation is an androgen-dependent disease, proliferative disease or amyloidosis;
[28] A method of treatment or prevention of conditions or disorders having sensitivity to selective androgen receptor modulation, comprising a step of administering a therapeutically effective amount of the compound according to any one of [1] to [20] to a subject in need of the treatment or prevention;
[29] The method according to [28], wherein the condition or disorder having sensitivity to selective androgen receptor modulation is an androgen-dependent disease, proliferative disease or amyloidosis; and
[30] The method according to [28], wherein the condition or disorder having sensitivity to selective androgen receptor modulation is a malignant tumor containing an androgen receptor of breast, brain, skin, ovary, bladder, lymph node, liver, kidney, uterus, spleen, endometrium, lung, colon or prostate; prostatic hypertrophy, ADAM, trichosis, acne, seborrhea, androgenic alopecia, excessive hair growth, libido regression, sexual dysfunction, muscular weakness, aorta smooth muscle cell proliferation, Alzheimer's disease, mad cow disease, new variant Creutzfeldt-Jacob disease, familial amyloid polyneuropathy, osteoporosis, dyslipidemia, obesity, diabetes mellitus, depression, urine incontinence, arterial sclerosis, uterine fibroid disease or endometriosis;
among others.

According to the present invention, provided are a novel thiophene derivative as SARM, its pharmaceutically acceptable salt, further, a pharmaceutical composition comprising these compounds as an active ingredient, which is useful for treatment and prevention of conditions or disorders having sensitivity to selective androgen receptor modulation; among others.

### MODE FOR CARRYING OUT THE INVENTION

The terms denote meanings generally understood in the art. The following definitions are used to explain the terms, and not construed to limit them.

The preferable number of atoms (for example, carbon) used in the entire specification is represented by, for example, "Cx-Cy", and this means that a specific number (x to y) of carbon atoms are contained. The same rule shall be applied also to other preferable terms and ranges. For example, "C1-C6 alkyl" represents an alkyl group containing 1 to 6 carbon atoms, "C2-C6 alkenyl" represents an alkenyl group containing 2 to 6 carbon atoms, "C2-6 alkynyl" represents an alkenyl group containing 2 to 6 carbon atoms, "C1-C6 alkylene" represents an alkylene group containing 1 to 6 carbon atoms, and "C3-C6 cycloalkyl" represents a cycloalkyl group containing 3 to 6 carbon atoms.

The term "alkyl" used in the present specification means a linear or branched saturated hydrocarbon containing a specific number of carbon atoms, and this may be substituted with a plurality of substituents included in the present invention, depending on the situation. Examples of such "alkyl" include C1-C6 alkyl, for example, methyl, ethyl, propyl, isopropyl, butyl, isobutyl, n-butyl, t-butyl, pentyl, isopentyl, n-pentyl, hexyl and the like.

The term "alkenyl" used in the present specification means a linear or branched aliphatic hydrocarbon having two or more carbon atoms and containing at least one carbon-carbon double bond, and this may be substituted with a plurality of substituents included in the present invention, depending on the situation. Examples of such "alkenyl" include C2-C6 alkenyl, for example, ethenyl, propenyl, butenyl, pentenyl, hexenyl and the like.

The term "alkynyl" used in the present specification means a linear or branched aliphatic hydrocarbon having two or more carbon atoms and containing at least one carbon-carbon triple bond, and this may be substituted with a plurality of substituents included in the present invention, depending on the situation. Examples of such "alkynyl" include C2-6 alkynyl, for example, ethynyl, propynyl, butynyl, pentynyl, hexynyl and the like.

The term "alkylene" used in the present specification means a linear or branched di-valent hydrocarbon group, and this may be substituted with a plurality of substituents included in the present invention, depending on the situation. Examples of such "alkylene" include C1-C6 alkylene, for example, methylene, ethylene, propylene, butylene, pentylene, hexylene and the like.

The term "cycloalkyl" used in the present specification means a non-aromatic cyclic hydrocarbon ring having a specific number of carbon atoms, and this may be substituted with a plurality of substituents included in the present invention, depending on the situation, and possibly, an alkylene linker is contained, and in this case, the cycloalkyl can be bonded via the linker. Examples of "cycloalkyl" include C3-C6 cycloalkyl, for example, cyclopropyl, cyclobutyl, cyclopentyl, cyclohexyl and the like. Cyclopropyl is preferable.

The term "halo" or "halogen," used in the present specification means fluorine, chlorine, bromine or iodine.

The term "haloalkyl" used in the present specification means the above-described alkyl group substituted with at least one halogen. Examples of "haloalkyl" include fluoromethyl, chloromethyl, difluoromethyl, trifluoromethyl and the like. Trifluoromethyl is preferable.

The term "hydroxy" used in the present specification means a -OH group.

The term "formyl" used in the present specification means a -C(O)H group.

The term "alkoxy" used in the present specification means a -ORa group, in which Ra represents the above-described alkyl. Examples of such "alkoxy" include C1-C6 alkoxy, for example, methoxy, ethoxy, propoxy, isopropoxy, butoxy, isobutoxy, sec-butoxy, tert-butoxy, pentoxy, hexoxy and the like.

The term "nitro" used in the present specification means a -NO₂ group.

The term "cyano" used in the present specification means a -CN group.

The term "optionally substituted (may be substituted)" used in the present specification means that a group such as alkyl, alkenyl, alkynyl, alkylene, cycloalkyl, haloalkyl or alkoxy and the like may be substituted with at least one substituent described in the present specification or may not be substituted. Examples of such a substituent include a halogen, hydroxy, alkyl, alkenyl, alkynyl, haloalkyl, cycloalkyl, alkoxy, formyl, cyano and the like.

The compound of the formula (I) can be crystallized in the form of two or more configurations, which is a known feature as polymorphism, and such polymorphic configurations (polymorph) are within the range of the formula (I). Polymorphism can generally occur as a reaction corresponding to a change in temperature or pressure or both. Polymorphism can occur also from a variation in a crystallization process. Polymorphism can be identified by various physical features known in the art such as X-ray diffraction pattern, degree of solubility and melting point.

The specific compound described in the present specification contains at least one chiral center, and can present in the form of a plurality of stereoisomers. A mixture of stereoisomers and, purified enantiomers, or an enantiomerically/diastereomerically-enriched mixture are also included in the present invention. Various isomers of a compound of the formula (I) and any completely or partially equilibrated mixtures thereof are also included in the present invention. Further included are various isomers of a compound of the formula (I), in the form of a mixture with correspondent isomers formed by inversion of at least one chiral center. Specific optical isomers can be separated and recovered by technologies known in the art such as chromatography in chiral stationary phase or chiral salt formation and subsequent separation by selective crystallization. Alternatively, by use of specific optical isomers as a starting material, correspondent isomers can be obtained as a final product.

Further, the compound of the formula (I) may be a crystal, and both a single crystal form and a mixture of crystal forms are included in the compound of the formula (I). The crystal can be produced by crystallization using a crystallization method known per se. Compounds labeled with isotopes (for example, ³H, ¹⁴C, ¹⁸F, ³⁵S, ¹²⁵I and the like) and the like are also included in the compound of the formula (I).

The term "salt" used in the present specification means a non-toxic salt of the inventive compound prepared from an inorganic acid or an organic acid. Preferably, the salt of the present invention is a pharmaceutically acceptable salt.

"Pharmaceutically acceptable" means that the salt is suitable for use in contact with human and animal tissues, without excessive toxicity, stimulation or other problems or complications, in proportion to suitable advantage/risk ratio, within the scope of sound medical judgment. The pharmaceutically acceptable salt can include acid addition salts. Typical salts include acetates, benzenesulfonates, benzoates, hydrogencarbonates, hydrogen sulfates, bitartarates, borates, bromides, edetates, camsilates, carbonates, chlorides, clavulanates, citrates, dihydrochlorides, edisylates, estolates, esylates, fumarates, gluceptates, gluconates, glutamates, glycollyl arsanilates, hexyl resorcinates, hydrabamines, hydrobromides, hydrochlorides, hydroxynaphthoates, iodides, isethionates, lactates, lactobionates, laurates, malates, maleates, mandelates, mesylates, methyl bromides, methyl nitrates, methyl sulfates, monopotassium malate, mucates, napsylates, nitrates, N-methylglucamine, oxalates, pamoates (embonates), palmitates, pantothenates, phosphates/diphosphates, polygalacturonates, potassium salts, salicylates, sodium salts, stearates, basic acetates, succinates, sulfates, tannates, tartrates, teoclates, tosylates, triethiodide, trimethylammonium and valerates. Pharmaceutically unacceptable other salts are also useful in production of the inventive compound in some cases, and these materials constitute another embodiment of the present invention.

The term "solvate" used in the present specification means a composite composed of a solute (in the case of the present invention, a compound of the formula (I) or its salt or physiologically functional derivative) and a solvent in various stoichiometric ratios. Such a solvent should not disturb the biological activity of the solute, in the object of the present invention. Non-limiting specific examples of suitable solvents include, but not limited to, water, methanol, ethanol and acetic acid. Preferably, the solvent to be used is a pharmaceutically acceptable solvent. Non-limiting specific examples of suitable pharmaceutically acceptable solvents include water, ethanol and acetic acid. Most preferably, the solvent to be used is water.

The term "physiologically functional derivative" used in the present specification means any pharmaceutically acceptable derivative of the inventive compound which can, when administered to mammals, directly or indirectly give the inventive compound or its active metabolite. For example, various ester derivatives which can be converted into the inventive compound by hydrolysis in blood, and the like, are listed. Reports regarding this method are disclosed in T. Higuchi and V. Stella, "Prodrugs as Novel Delivery Systems" Vol.14 of the A.C.S. Symposium Series, and Bioreversible Carriers in Drug Design, ed. Edward B. Roche, American Pharmaceutical Association and Pergamon Press, 1987, and these are incorporated as a part of the present specification by reference.

The term "effective amount" used in the present specification means the amount of a medicinal substance or medicinal agent causing, for example, a biological or medical response of tissue, animal or human, sought by researchers or clinicians. The biological or medical response can be regarded as a preventive response or therapeutic response. The term "therapeutically effective amount" means any amount causing treatment, cure, prevention or alleviation improving diseases, disorders or side effects, or causing retardation of the progress speed of diseases or disorders, as compared with subjects not underwent administration of such an amount. This term includes in its range also an amount effective for promoting a normal physiological function. In remedy use, a therapeutically effective amount of a compound of the formula (I) and its salt, solvate and physiologically functional derivative can be administered as a compound raw material. Without particular limitation, the effective amount of a compound of the formula (I) is usually 0.1 to 100 mg/kg body weight per day.

The compound of the formula (I) or its salt, solvate or physiologically functional derivative can be provided in the form of a pharmaceutical composition.

In one embodiment, the present invention provides a pharmaceutical composition comprising an effective amount of a compound of the formula (I) or its salt, solvate or physiologically functional derivative described in the present specification, and at least one pharmaceutically acceptable carrier, diluent or excipient. The carrier, diluent or excipient should be a material acceptable in that it is compatible with other components in the preparation and it is not harmful on the subject of the pharmaceutical composition.

In another embodiment, the present invention provides use of the inventive compound in production of a medicine for use in treatment or prevention of androgen-dependent diseases (for example, prostatic hypertrophy, ADAM, trichosis, acne, seborrhea, androgenic alopecia, excessive hair growth, libido regression, sexual dysfunction, muscular weakness and the like), proliferative diseases (for example, malignant tumors containing an androgen receptor of breast, brain, skin, ovary, bladder, lymph node, liver, kidney, uterus, spleen, endometrium, lung, colon or prostate; aorta smooth muscle cell proliferation and the like), amyloidosis (for example, Alzheimer's disease, mad cow disease, new variant Creutzfeldt-Jacob disease, familial amyloid polyneuropathy and the like), and other androgen receptor (AR)-associated diseases (for example, osteoporosis, dyslipidemia, obesity, diabetes mellitus, depression, urine incontinence, arterial sclerosis, uterine fibroid disease, endometriosis and the like).

The inventive compound is an AR modulator which modulates the function of an androgen receptor (AR) as a nuclear hormone receptor. The inventive compound is useful for treatment of AR-associated diseases or conditions such as diseases or conditions which are prevented, alleviated or cured by modulation of the function or activity of AR. Such modulation can be limited in certain tissue, or can spread in the whole body of the subject undergoing therapy.

In a further embodiment, the present invention provides use of the inventive compound for treatment or prevention of various diseases including, but not limited to, treatment or prevention of osteoarthropathy and osteochondrodysplasia; treatment or prevention of Paget's disease, age-related functional decline (ARFD), dry eye, sarcopenia, cachexia, chronic fatigue syndrome, acute fatigue syndrome and chronic muscle pain; recovery of AIDS, burn injury and external injury; treatment or prevention of thrombocytopenia, anemia, irritable bowel syndrome, cardiovascular disease, cardiac dysfunction, congestive cardiac failure and hypertension; treatment or prevention of hyperinsulinemia, insulin resistance and arterial sclerosis; treatment or prevention of nervousness and hypersensitivity; betterment of cognition function; treatment or prevention of polycystic ovary syndrome; treatment or prevention of contraception; treatment or prevention of tuberculosis, Hansen's disease, familial Mediterranean fever, bronchiectasis, Muckle-Wells syndrome, reactive AA amyloidosis, immunocyte-associated amyloidosis and osteomyelitis; treatment or prevention of Down syndrome, cerebrovascular amyloidosis, genetic amyloid brain hemorrhage, Gerstmann-Sträussler-Scheinker syndrome, transmissible spongiform encephalopathy, scrapie, adult-onset diabetes mellitus, skin amyloidosis and focal nodular amyloidosis; and treatment or prevention of senile amyloidosis and dialysis amyloidosis.

In still another embodiment, the present invention provides a production method of a pharmaceutical composition, comprising mixing a compound of the formula (I) or its salt, solvate or physiologically functional derivative with at least one pharmaceutically acceptable carrier, diluent or excipient.

The inventive compound can be produced by various methods including known standard synthesis methods. In the following schemes, R¹, R² , R³, R⁴, R⁵, R⁸, R⁹ and R¹⁰ are as defined in the present specification, unless otherwise stated. Exemplary general synthesis methods are shown below.

In the following schemes, protective groups for sensitive or reactive groups are used, if necessary, according to the general theory of synthetic chemistry. The protective groups are used according to standard methods of organic synthesis (t. W. Green and P. G. M. Wuts (1991), Protecting Groups in Organic Synthesis, John Wiley & Sons). These groups are easily removed in a compound synthesis stage, using methods known in the art. Selection of the methods and reaction conditions and orders in operation thereof correspond to those in production of a compound of the formula (I).

An aminothiophene derivative which is an intermediate necessary for synthesis of a compound of the formula (I) can be produced by the following method (scheme 1). Electron-deficient 2-formylthipphene can be reacted with a secondary amine such as N-(cyclopropylmethyl)-N-propylamine and the like in a solvent such as water and the like, in the presence of a base such as triethylamine and the like, to produce the correspondent aminothiophene derivative. Further, electron-deficient 2-cyanothiophene can be reacted with a primary amine such as propylamine and the like, in the presence of a base such as sodium hydrogen carbonate and the like, or under heating conditions without solvent, to produce the correspondent aminothiophene derivative. Furthermore, the correspondent aminothiophene derivative can be produced by direct amidation in the presence of a catalyst such as copper(I) iodide and the like.

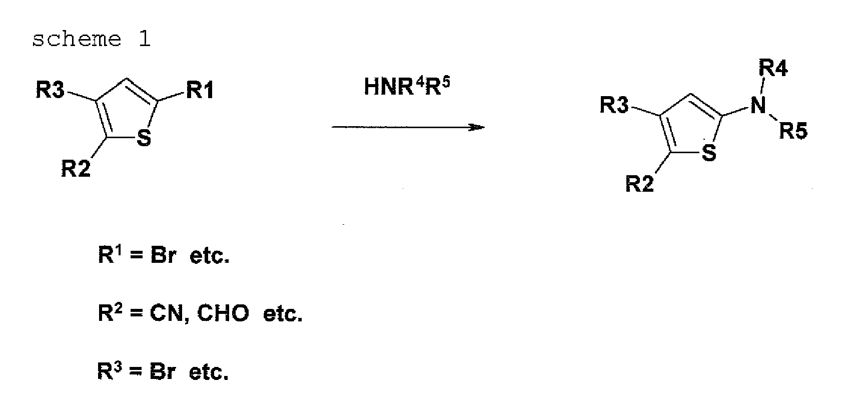

The intended compound tertiary aminothiophene derivative can be produced by reacting secondary aminothiophene with an alkyl halide R⁵-X such as propyl iodide and the like, in the presence of a base such as sodium hydride and the like (scheme 2). Likewise, it can be produced by alkylating secondary aminothiophene using an α-haloester such as ethyl bromoacetate and the like. Further, the correspondent amide derivative can be produced by treating secondary aminothiophene with an acid chloride such as acetyl chloride and the like.

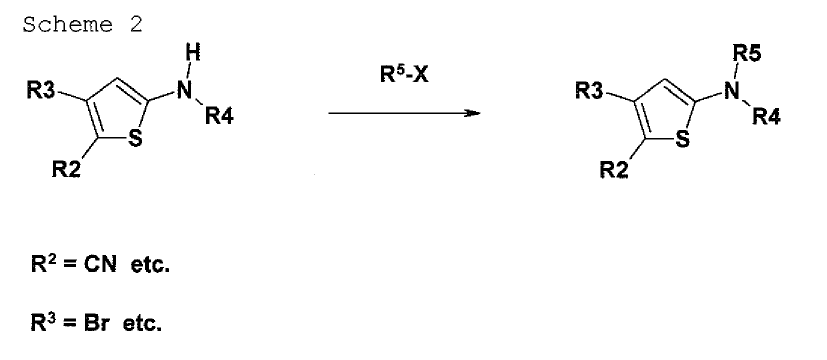

An acetate derivative can be saponified to obtain the correspondent carboxylic acid, then, this can be converted into an acid chloride before coupling with various amines, to produce the correspondent amide derivative (scheme 3, method A). Further, an acetaldehyde derivative can be reacted with an alkylating agent such as methylmagnesium bromide and the like to produce the correspondent tertiary alcohol form (scheme 3, method B).

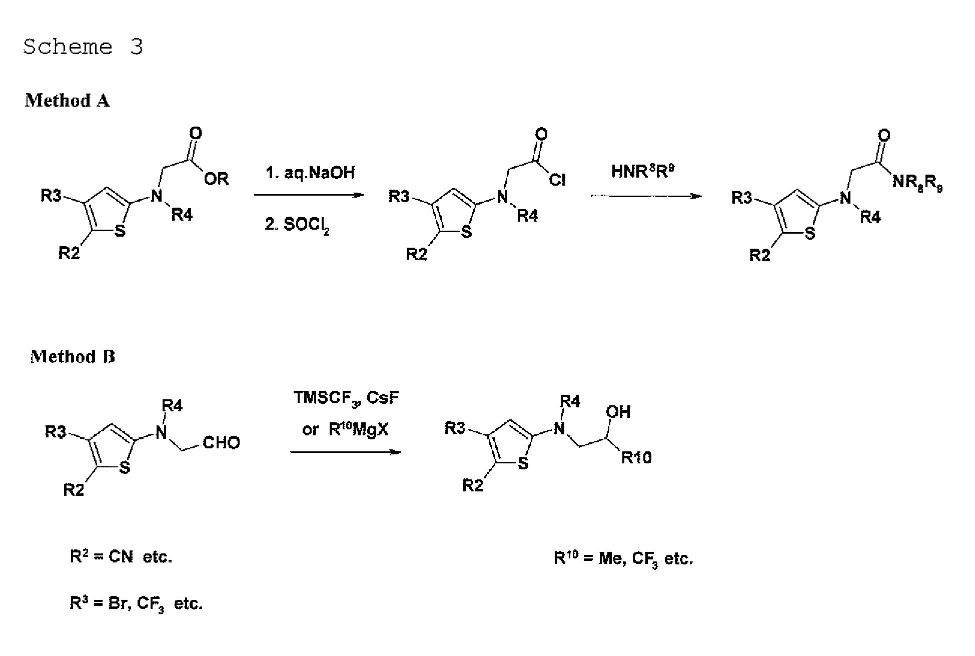

The inventive typical androgen receptor modulator compounds, agonists, partial agonists, antagonists and partial antagonists include the following compounds:
3-bromo-5-[(cyclopropylmethyl)(propyl)amino]thiophene-2-carbaldehyde;
3-bromo-5-[(cyclopropylmethyl)(propyl)amino]thiophene-2-carbonitrile;
3-chloro-5-[(cyclopropylmethyl)(propyl)amino]thiophene-2-carbonitrile;
5-[(cyclopropylmethyl)(propyl)amino]-2-(trifluoromethyl)thiophene-2-carbaldehyde;
5-[(cyclopropylmethyl)(propyl)amino]-3-(trifluoromethyl)thiophene-2-carbonitrile;
2-bromo-5-[(cyclopropylmethyl)(propyl)amino]thiophene-3-carbonitrile;
5-[(cyclopropylmethyl)(propyl)amino]-2-(trifluoromethyl)thiophene-3-carbonitrile;
3-bromo-5-(pyrrolidin-1-yl)thiophene-2-carbonitrile;
3-bromo-5-(2-methylpiperidin-1-yl)thiophene-2-carbonitrile;
3-bromo-5-(piperidin-1-yl)thiophene-2-carbonitrile;
3-bromo-5-(3,5-dimethylpiperidin-1-yl)thiophene-2-carbonitrile;
3-bromo-5-(4-methylpiperidin-1-yl)thiophene-2-carbonitrile;
3-bromo-5-[ (methyl)(2-methylpropyl)amino]thiophene-2-carbonitrile;
3-bromo-5- (dipropylamino)thiophene-2-carbonitrile;
3-bromo-5- (diallylamino)thiophene-2-carbonitrile;
5-[bis(2-methoxyethyl)amino]-3-bromothiophene-2-carbonitrile;
3-bromo-5-(2-ethylpiperidin-1-yl)thiophene-2-carbonitrile;
3-bromo-5-(2-propylpiperidin-1-yl)thiophene-2-carbonitrile;
N-(4-bromo-5-cyanothiophen-2-yl)-N-propylacetamide;
N-(4-bromo-5-cyanothiophen-2-yl)-N-propylpropanamide;
N-(4-bromo-5-cyanothiophen-2-yl)-N-propylbutanamide;
N-(4-bromo-5-cyanothiophen-2-yl)-2-methyl-N-propylpropanamide;
3-bromo-5-[(propyl)(2,2,2-trifluoroethyl)amino]thiophene-2-carbonitrile;
N-(4-bromo-5-cyanothiophen-2-yl)-N-propylglycine ethyl ester;
N2-(4-bromo-5-cyanothiophen-2-yl)-N-methyl-N2-propylglycinamide;
N-(4-bromo-5-cyanothiophen-2-yl)-N-propylglycine;
N2-(4-bromo-5-cyanothiophen-2-yl)-N2-propylglycinamide;
3-bromo-5-[(cyanomethyl)(propyl)amino]thiophene-2-carbonitrile;
N2-(4-bromo-5-cyanothiophen-2-yl)-N,N-dimethyl-N2-propylglycinamide;
3-bromo-5-[(1,3-dioxolan-2-ylmethyl)amino]thiophene-2-carbonitrile;
3-bromo-5-[(1,3-dioxolan-2-ylmethyl)(propyl)amino]thiophene-2-carbonitrile;
3-bromo-5-[(2-oxoethyl)(propyl)amino]thiophene-2-carbonitrile;
3-bromo-5-[propyl(3,3,3-trifluoro-2-hydroxypropyl)amino]thiophene-2-carbonitrile;
3-bromo-5-[(2-hydroxypropyl)(propyl)amino]thiophene-2-carbonitrile;
3-bromo-5-(2-ethyl-6-oxopiperidin-1-yl)thiophene-2-carbonitrile;
3-bromo-5-(2-ethyl-5-thioxopiperidin-1-yl)thiophene-2-carbonitrile;
N-(4-bromo-5-cyanothiophen-2-yl)-N-propylpropanthioamide;
3-bromo-5-[2-(hydroxymethyl)piperidin-1-yl]thiophene-2-carbonitrile;
3-bromo-5-(2-formylpiperidin-1-yl)thiophene-2-carbonitrile;
3-bromo-5-[2-(2,2,2-trifluoro-l-hydroxyethyl)piperidin-1-yl]thiophene-2-carbonitrile;
3-bromo-5-[2-(1-hydroxyethyl)piperidin-1-yl]thiophene-2-carbonitrile;
3-bromo-5-{[2-(hydroxyimino)ethyl](propyl)amino}thiophene-2-carbonitrile;
5-[2-(2,2,2-trifluoro-1-hydroxyethyl)piperidin-1-yl]-3-(trifluoromethyl)thiophene-2-carbonitrile;
5-[(propyl)(2,2,2-trifluoroethyl)amino]-3-trifluoromethylthiophene-2-carbonitrile;
5-[(propyl)(2,2,2-trifluoroethyl)amino]-3-methylthiophene-2-carbonitrile; and
5-(2-cyanopiperidin-1-yl)-3-bromo-thiophene-2-carbonitrile.

The therapeutically effective amount of the inventive compound is influenced by a lot of factors. For example, all of the sex, age and body weight of the subject, strict conditions needing treatment and severity level thereof, natures of the preparation and administration route thereof are factors to be considered. The therapeutically effective amount should be finally judged by an attending physician or veterinary doctor. Independently, the effective amount of a compound of the formula (I) to treat a debilitated human is, in general, 0.1 to 100 mg per kg of the body weight of the subject (mammal) per day. More generally, the effective amount is 1 to 10 mg/kg body weight per day. Therefore, the actual amount per day will be usually 70 to 700 mg in the case of an adult mammal of 70 kg. This amount can be administered in the form of single dose per day or in the form of divided amounts for several doses (for example, 2 to 5, or more) per day so that the total daily dose is identical. The effective amount of its salt, solvate or physiologically functional derivative can be determined relatively base on the effective amount of a compound of the formula (I) itself. Also for treatment or prevention of other conditions referred in the present specification, the same dosage is believed to be suitable.

A medicinal preparation can be provided as a unit dosage form containing a prescribed amount of active ingredients per unit administration. Such a unit can contain a compound of the formula (I) in an amount of 0.5 mg to 1 g according to conditions to be treated, the administration route and the age, body weight and conditions of the patient, as a non-limiting example. A preferable unit administration preparation contains the above-described daily dose or divided amount of active ingredients, or a suitable proportion thereof. Such a medicinal preparation can be produced by conventional methods in the preparation technical field, for example, methods described in Japanese Pharmacopoeia, and the like.

A medicinal preparation is suitable for administration via any suitable routes, for example, oral (including buccal or sublingual), rectal, intranasal, local (including buccal, sublingual or transdermal), vaginal or parenteral (including subcutaneous, intramuscular, intravenous or intradermal) routes.

Examples of medicinal preparations suitable for oral administration include tablets (including sugar-coated tablet, film-coated tablet, sublingual tablet, or orally-disintegrating tablet), capsules (including soft capsule, ormicrocapsule), granule, powder, troche, liquid or suspensions (each including aqueous or non-aqueous liquid), edible foam or whip, or oil-in-water type liquid emulsion or water-in-oil type liquid emulsion, and the like These preparations may be controlled release formulations (for example, extended release microcapsule) such as quick release formulations or extended release formulations and the like.

In the case of oral administration in the form of a tablet or capsule, active drug components can be combined with pharmaceutically acceptable oral nontoxic inert carriers (for example, ethanol, glycerol, water and the like).

The powder can be produced, in general, by powdering the compound into suitable minute size, and mixing with suitable pharmaceutical carriers, for example, edible carbohydrates (for example, starch or mannitol). Flavoring agents, preserving agents, dispersing agents and coloring agents may be contained.

The capsule can be produced by preparing a powder, liquid or suspension mixture, and performing encapsulation thereof with gelatin or some other suitable shell materials. Before encapsulation, glidants and lubricants (for example, colloidal silica, talc, magnesium stearate, calcium stearate or solid polyethylene glycol) can be added to the mixture. For improving the bioavailability of a medicine when a capsule is ingested, disintegrating agents or solubilizing agents (for example, agar, calcium carbonate or sodium carbonate) can also be added. Further, depending on the situation or if necessary, suitable binding agents, lubricants, disintegrating agents and coloring agents may be added into the mixture.

Examples of the binding agent include starch, gelatin, natural sugars (for example, glucose or β-lactose), corn sweetening agent, natural and synthetic rubbers (for example, acacia, tragacanth or sodium alginate), carboxymethyl cellulose, polyethylene glycol, wax and the like. Examples of the lubricant include sodium oleate, sodium stearate, magnesium stearate, sodium benzoate, sodium acetate, sodium chloride and the like. Examples of the disintegrating agent include starch, methyl cellulose, agar, bentonite, xanthan gum and the like.

For preparing tablets, for example, a powder mixture is prepared and granulated or dry-compressed into a granule, a lubricant and a disintegrating agent are added thereto, and the mixture is compressed into a tablet. The powder mixture can be produced by mixing the compound (suitably, powdered compound) with a diluent or base. The components which can be used depending on the situation include binding agents (for example, carboxymethyl cellulose, alginate, gelatin or polyvinylpyrrolidone), dissolution retardants (for example, paraffin), reabsorption promoters (for example, quaternary salt) and/or absorbing agents (for example, bentonite, kaolin or dicalcium phosphate). The powder mixture can be wet-granulated with a solution of a binding agent, for example, syrup, starch paste, acadia viscous substance or cellulose, or a polymerizable substance, and sieved. As an alternative means for granulation, a powder mixture can be processed in a tablet machine, and the material to be generated is incompletely formed slag which is then decomposed into granules. For prevention of adhesion to a tablet forming mold, the granule can be lubricated by addition of stearic acid, stearate, talc or mineral oil. Subsequently, the lubricated mixture is compressed into a tablet. Further, it is also possible to combine the inventive compound with a free-flowable inert carrier, and compress the mixture directly into a tablet, via no granulation or dry granule compressing process. It is possible to provide a transparent or opaque protective coating composed of a shellac sealing coat, sugar or polymer material coating, and wax gloss coating. For discriminating different unit dosage forms, it is possible to add a dye to these coatings.

Oral liquid formulations, for example, liquid, syrup and elixir can be produced as a unit dosage form so that given amount thereof contains a prescribed amount of the compound. The syrup can be produced, for example, by dissolving the compound in a suitably flavored aqueous solution. In contrast, the elixir can be produced by using a nontoxic alcoholic vehicle. The suspension can be formulated, in general, by dispersing the compound in a nontoxic vehicle. Stabilizing agents and emulsifying agents (for example, ethoxylated isostearyl alcohol and polyoxyethylene sorbitol ether), preserving agents; flavoring additives (for example, peppermint oil) or natural sweeteners, saccharin or other artificial sweeteners, and the like may also be added.

The compound of the formula (I) and its salt, solvate and physiologically functional derivative can be administered in the form of a liposome delivery system (for example, small unilamillar vesicle, large unilamillar vesicle and multilamillar vesicle). A liposome can be formed from various phospholipids (for example, cholesterol, stearylamine or phosphatidylcholine).

The compound of the formula (I) and its salt, solvate and physiologically functional derivative can also be delivered by use of a monoclonal antibody as an individual carrier to which molecules of the compound are bonded.

Further, the compound can be bonded to a suitable polymer as a targetable drug carrier. Such a polymer includes polyvinylpyrrolidone (PVP), pyran copolymer, polyhydroxypropylmethacrylamide-phenol, polyhydroxyethyl-aspartamidephenol or, polyethylene oxide polylysine substituted with a palmitoyl residue. Further, the compound can be bonded to biodegradable polymers useful for accomplishment of controlled release of a drug, for example, polylactic acid, polyepsiloncaprolactone, polyhydroxybutyric acid, polyorthoester, polyacetal, polydihydropyrane, polycyano acrylate, and hydro gel cross-linked or amphipathic block polymer.

The medicinal preparation suitable for transdermal administration can be provided as separate patches intended to keep close contact with the skin of the subject for a long period of time. For example, active ingredients can be delivered from the patch by iontophoresis as described generally in Pharmaceutical Research, 3(6), 318(1986).

The medicinal preparation suitable for local administration can be formulated in the form of an ointment, cream, suspension, lotion, powder, liquid, paste, gel, spray, aerosol or oil.

For treatment of eyes or other external tissues, for example, mouth and skin, the preparation can be applied as a local ointment or cream. If the case of formulation into an ointment, active ingredients can be used together with paraffinic or water-miscible ointment bases. Alternatively, active ingredients can be formulated into a cream with an oil-in-water type cream base or a water-in-oil type base.

The medicinal preparations suitable for local administration to eyes include eye drops containing active ingredients dissolved or suspended in a suitable carrier, particularly, an aqueous solvent.

The medicinal preparations suitable for local administration to mouth cavity include lozenge, troche and mouthwash.

The medicinal preparations suitable for intranasal administration, when the carrier is solid, include, for example, coarse powders having a particle size of 20 to 500 microns. The powder can be administered by using an inhalant, that is, by quick inhalation by intranasal pass from a powder vessel closely held to nose. Suitable preparations, when the carrier is liquid, for administration as a nasal spray or a nasal drop include an aqueous or oily solution of active ingredients.

The medicinal preparations suitable for administration by inhalation include fine particle dust formulations or mists, and these can be generated by various types of pressurized metered-dose aerosols, nebulizers or insufflators.

The medicinal preparations suitable for rectal administration can be provided as a suppository or an enema.

The medicinal preparations suitable for vaginal administration can be provided as a pessary, tampon, cream, gel, pasta, foam or spray.

The medicinal preparations suitable for parenteral administration include aqueous or non-aqueous sterile injection solutions which can contain an antioxidant, a buffering agent, a bacteriostatic agent, and a solute rendering the preparation isotonic against the blood of the intended subject; and aqueous or non-aqueous sterile suspensions which can contain a suspending agent and a thickener. The preparation can be provided in the form of a vessel for single administration or multiple administration, for example, a sealed ample and a vial, and can be preserved in lyophilized condition only needing addition of a sterile liquid carrier, for example, injection water, directly before use. The injection solution and suspension to be blended if necessary can be prepared from a sterile powder, granule and tablet.

The preparation can contain other substances which are usual in the art regarding the type of the preparation, in addition to the components listed above. For example, the preparations suitable for oral administration can contain a flavoring agent and a coloring agent.

The inventive compound can be used singly, or in combination with other therapeutic agents to treatment of the above-described conditions. As an example, the combined remedy for osteoporosis of the present invention can include administration of at least one compound of the formula (I) or its salt, solvate or physiologically functional derivative, and use of at least one other osteoporosis remedy. As another example, the combined remedy of the present invention includes administration of at least one compound of the formula (I) or its salt, solvate or physiologically functional derivative, and at least one other osteoporosis therapeutic agent, for example, an anti-bone resorbing agent. A compound of the formula (I) and other pharmaceutically active substance can be administered together or separately, and when administered separately, these can be administered simultaneously or administered continuously in any order. The amounts and relative administration moments of a compound of the formula (I) and other pharmaceutically active substance are selected so that the desire combined remedy effect is attained. The combined administration of a compound of the formula (I) or its salt, solvate or physiologically functional derivative, and other therapeutic agent can be a combination of appendant administration using (1) a single pharmaceutical composition containing both compounds or (2) separate pharmaceutical compositions containing one of them and the other respectively. Alternatively, the combined agents can be administered separately and continuously, an in this case, one therapeutic agent is administered, then, another agent is administered (the reverse order is also possible). Such continuous administration operations may be near time-wise or away time-wise.

Another potential osteoporosis therapeutic agent is an osteogenesis (anabolic) agent. The osteogenesis agent can induce an increase in a parameter such as bone mineral density which is larger than an increase attainable with an anti-resorbing agent. In some cases, such an anabolic agent increases a column binding property, and can cause an increase in bone structural integrity.

Other potential therapeutic combinations include a combination of the compound of the present invention with other compound of the present invention, growth promoting agent, growth hormone secretagogue, growth hormone releasing factor and analogs thereof, growth hormone and analogs thereof, somatomedin, α-adrenergic agonist, serotonin 5-HT_{D} agonist, somatostatin or substance suppressing release thereof, 5α-reductase inhibitor, aromatase inhibitor, GnRH agonist or antagonist, parathyroid hormone, bisphosphonate, estrogen, testosterone, SERM, progesterone receptor agonist or antagonist, and/or other modulator of nuclear hormone receptor.

The compounds included in the present invention are used as a selective agonist, partial agonist and antagonist, and compounds having a mixed steroid activity can also be used.

The inventive compound can be used in treatment of various disorders and conditions, and the inventive compound can also be used for such treatment or prevention in combination with other various suitable therapeutic agents which are useful for treatment or prevention of these disorders or conditions. Non-limiting specific examples thereof include a combination of the present invention with an anti-diabeteic drug, anti-osteoporosis drug, anti-obesity drug, anti-inflammatory drug, anti-anxiety drug, antidepressant, antihypertensive, antiplatelet drug, antithrombotic and thrombolytic, cardiac glycoside, cholesterol or lipid lowering drug, mineral corticoid receptor antagonist, phosphodiesterase inhibitor, kinase inhibitor, thyroid mimic, anabolic agent, virus therapy, cognitive impairment therapy, sleep disorder therapy, sexual dysfunction therapy, contraceptive agent, cytotoxic agent, radiation therapy, antiproliferative agent and antitumor agent. Further, the inventive compound can be combined with nutritious supplements, for example, an amino acid, triglyceride, vitamin, mineral, creatine, piloic acid, carnitine or coenzyme Q10.

Another embodiment of the present invention is use of the inventive compound for treatment or prevention of various disorders including, but not limited to, treatment or prevention of conditions or disorders having sensitivity to selective androgen receptor modulation, preferably treatment or prevention of androgen-dependent diseases, proliferative diseases or amyloidosis, more preferably treatment or prevention of prostatic hypertrophy, ADAM, trichosis, acne, seborrhea, androgenic alopecia, excessive hair growth, libido regression, sexual dysfunction, muscular weakness; malignant tumors containing an androgen receptor of breast, brain, skin, ovary, bladder, lymph node, liver, kidney, uterus, spleen, endometrium, lung, colon or prostate; aorta smooth muscle cell proliferation, Alzheimer's disease, mad cow disease, new variant Creutzfeldt-Jacob disease, familial amyloid polyneuropathy, osteoporosis, dyslipidemia, obesity, diabetes mellitus, depression, urine incontinence, arterial sclerosis, uterine fibroid disease or endometriosis.

Particularly, the inventive compound is useful for treatment or prevention of conditions or disorders having sensitivity to selective androgen receptor modulation, the compound being used singly or in combination with other substances. Preferably, the condition or disorder having sensitivity to selective androgen receptor modulation is an androgen-dependent disease, proliferative disease or amyloidosis. More preferably, the condition or disorder having sensitivity to selective androgen receptor modulation is prostatic hypertrophy, ADAM, trichosis, acne, seborrhea, androgenic alopecia, excessive hair growth, libido regression, sexual dysfunction, muscular weakness; malignant tumor containing an androgen receptor of breast, brain, skin, ovary, bladder, lymph node, liver, kidney, uterus, spleen, endometrium, lung, colon or prostate; aorta smooth muscle cell proliferation, Alzheimer's disease, mad cow disease, new variant Creutzfeldt-Jacob disease, familial amyloid polyneuropathy, osteoporosis, dyslipidemia, obesity, diabetes mellitus, depression, urine incontinence, arterial sclerosis, uterine fibroid disease or endometriosis.

The marks and conventional practices used in the present specification correspond to those used in modern scientific literatures, for example, Journal of the American Chemical Society or Journal of Biological Chemistry. Particularly, the following abbreviations can be used in examples:
g (gram); mg (milligram); L (liter); mL (milliliter); M (molar concentration); mM (millimolar concentration); mol (mole); mmol (millimole); N (normal concentration); min (minute); h (hour); Hz (hertz); mp (melting point); eq (equivalent); PTLC (preparative thin layer chromatography); t_{R} (retention time); THF (tetrahydrofuran); DMF (N,N-dimethylformamide); NMP (1-methyl-2-pyrrolidinone); CDCl₃ (deuterated chloroform); LDA (lithiumdiisobutylamide); MeMgBr (methylmagnesium bromide); TFA (trifluoroacetic acid); NaHCO₃ (sodium hydrogen carbonate); Na₂CO₃ (sodium carbonate); Na₂SO₄ (sodium sulfate); Ma₂SO₄ (magnesium sulfate); HCl (hydrochloric acid); NaOH (sodium hydroxide).

The following analysis instruments were used to measure various data.
¹H-NMR: AV300M (manufactured by BRUKER)
GC-MS: QP-5000 (manufactured by Shimadzu Corporation)
LC-MS: LCQ (manufactured by Thermo Electron Corporation)

For ¹H-NMR spectrum, chemical shift is expressed in terms of ppm (ppm, δ value) and coupling constant is expressed in terms of hertz (Hz, J value). The separation parameter shows apparent multiplicity, and expressed as s (singlet), d (doublet), t (triplet), q (quartet), m (multiplet) or br (broad).

### EXAMPLES

The present invention will be illustrated further in detail by examples, reference examples and test examples mentioned below, but the present invention is not limited to them.

### Example 1

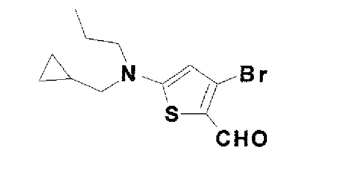

### (1) 3,5-dibromothiophene-2-carbaldehyde

A 2M LDA solution (24 mL, 48 mmol) and THF (20 mL) were added, and under nitrogen atmosphere, a THF solution (20 mL) of 2-bromothiophene (7.8 g, 48 mmol) was dropped at room temperature and the mixture was stirred for about 30 minutes. After cooling with dry ice/acetone, a THF solution (5 mL) of DMF (1.8 g, 0.5 eq) was added. The mixture was stirred for a while, then, poured into 1N HCl (250 mL), and extracted with ethyl acetate. The organic layer was washed with water, dried over Na₂SO₄, then, concentrated under reduced pressure to obtain a residue, which was purified by silica gel column chromatography (eluent: hexane/ethyl acetate = 20/1) to obtain the title compound (4.16 g, 85%) as pale yellow crystals. ¹H-NMR (300 MHz, CDCl₃): δ 7.15 (s, 1H), 9.83 (s, 1H)

### (2) 3-bromo-5-[(cyclopropylmethyl)(propyl)amino]thiophene-2-carbaldehyde

3,5-dibromothiophene-2-carbaldehyde (4.15 g, 15.3 mmol), N-(cyclopropylmethyl)-N-propylamine (2.26 g, 1.3 eq), triethylamine (3.10 g, 2.0 eq) and water (40 mL) were added, and under nitrogen atmosphere, the mixture was refluxed with heating for 18 hours. After cooling, the mixture was extracted with ethyl acetate, and the organic layer was washed with water, dried over Na₂SO₄, then, concentrated under reduced pressure to obtain a residue, which was purified by silica gel column chromatography (eluent: hexane/ethyl acetate = 6/1) to obtain the title compound (2.15 g, 46%) as a red orange oil.
¹H-NMR (300 MHz, CDCl₃): δ 0.26-0.31 (m, 2H), 0.59-0.66 (m, 2H), 0.95 (t, J = 7.5 Hz, 3H), 1.01-1.09 (m, 1H), 1.65-1.75 (m, 2H), 3.22 (d, J = 6.9 Hz, 2H), 3.37 (t, J = 7.8 Hz, 2H), 5.94 (s, 1H), 9.57 (s, 1H)
GC-MS(EI): m/z 301 (M), 303 (M+2)

### Example 2

### 3-bromo-5-[(cyclopropylmethyl)(propyl)amino]thiophene-2-carbonitrile

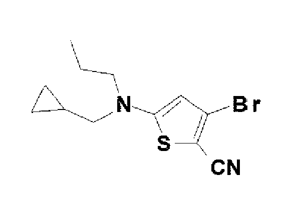

3-bromo-5-[(cyclopropylmethyl)(propyl)amino]thiophene-2-carbaldehyde (0.73 g, 2.42 mmol), hydroxylammonium sulfate (0.24 g, 1.2 eq) and NMP (5 mL) were added, and the mixture was heated at 120°C for 2.5 hours. After cooling, the solution was adjusted to alkaline (pH = about 9) with a 1N sodium hydroxide aqueous solution, extracted with ethyl acetate, washed with water, and dried over Na₂SO₄. The dried mixture was concentrated under reduced pressure to obtain a residue, which was purified by silica gel column chromatography (eluent: hexane/ethyl acetate = 8/1) to obtain the title compound (0.26 g, 36%) as a pale brown oil.
¹H-NMR (300 MHz, CDCl₃): 5 0.24-0.29 (m, 2H), 0.59-0.65 (m, 2H), 0.94 (t, J = 7.5 Hz, 3H), 1.04-1.14 (m, 1H), 1.61-1.74 (m, 2H), 3.15 (d, J = 6.6 Hz, 2H), 3.28 (t, J = 7.8 Hz, 2H), 5.77 (s, 1H)
GC-MS(EI): m/z 298 (M), 300 (M+2)

### Example 3

### 3-chloro-5-[(cyclopropylmethyl)(propyl)amino]thiophene-2-carbonitrile

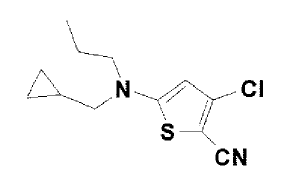

3-bromo-5-[(cyclopropylmethyl)(propyl)amino]thiophene-2-carbaldehyde (0.56 g, 1.85 mmol), hydroxylammonium hydrochloride (0.15 g, 1.2 eq) and NMP (3 mL) were added, and the mixture was heated at 110°C for 12 hours. After cooling, the solution was adjusted to alkaline (pH = about 9) with a 1N sodium hydroxide aqueous solution, extracted with ethyl acetate, washed with water, and dried over Na₂SO₄. The dried mixture was concentrated under reduced pressure to obtain a residue, which was purified by silica gel column chromatography (eluent: hexane/ethyl acetate = 8/1) to obtain the title compound (58 mg, 11%) as a pale yellow oil.
¹H-NMR (300 MHz, CDCl₃): 5 0.26-0.30 (m, 2H), 0.59-0.65 (m, 2H), 0.94 (t, J = 7.5 Hz, 3H), 1.03-1.12 (m, 1H), 1.64-1.72 (m, 2H), 3.15 (d, J = 6.6 Hz, 2H), 3.30 (t, J = 7.8 Hz, 2H), 5.73 (s, 1H)
GC-MS(EI): m/z 254 (M), 256 (M+2)

### Example 4

### 5-[(cyclopropylmethyl)(propyl)amino]-3-(trifluoromethyl)thiophene-2-carbaldehyde

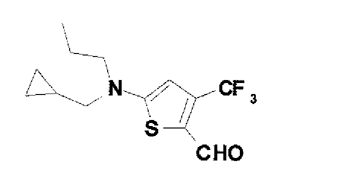

3-bromo-5-[(cyclopropylmethyl)(propyl)amino]thiophene-2-carbaldehyde (0.70 g, 2.34 mmol), methyl fluorosulfonyldifluoroacetate (0.90 g, 2.0 eq), copper(I) iodide (47 mg, 10 mol%) and DMF (4 mL) were added, and the mixture was heated at 120°C for 6 hours. To the cooled reaction mixture was added ethyl acetate and water, the organic layer was separated, then, washed with water, dried over Na₂SO₄, concentrated under reduced pressure to obtain a residue, which was purified by silica gel column chromatography (eluent: hexane/ethyl acetate = 10/1) to obtain the title compound (0.13 g, 19%) as a dark orange oil.
¹H-NMR (300 MHz, CDCl₃): δ 0.28-0.33 (m, 2H), 0.61-0.67 (m, 2H), 0.97 (t, J = 7.4 Hz, 3H), 1.09-1.14 (m, 1H), 1.67-1.77 (m, 2H), 2.27 (d, J = 6.6 Hz, 2H), 3.41 (t, J = 7.8 Hz, 2H), 6.13 (s, 1H), 9.74 (d, J = 0.9 Hz, 1H)
GC-MS(EI): m/z 291 (M)

### Example 5

### 5-[(cyclopropylmethyl)(propyl)amino]-3-(trifluoromethyl)thiophene-2-carbonitrile

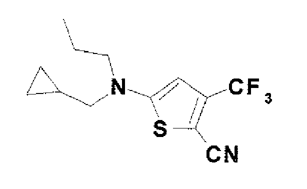

The title compound was obtained according to Example 2, using 5-[(cyclopropylmethyl)(propyl)amino]-3-(trifluoromethyl)thiophene-2-carbaldehyde instead of 3-bromo-5-[(cyclopropylmethyl)(propyl)amino]thiophene-2-carbaldehyde.
¹H-NMR (300 MHz, CDCl₃): δ 0.26-0.31 (m, 2H), 0.60-0.67 (m, 2H), 0.96 (t, J = 7.5 Hz, 3H), 1.03-1.10 (m, 1H), 1.66-1.74 (m, 2H), 3.19 (d, J = 6.6 Hz, 2H), 3.35 (t, J = 7.6 Hz, 2H), 5.95 (s, 1H)
GC-MS (EI) : m/z 288 (M)

### Example 6

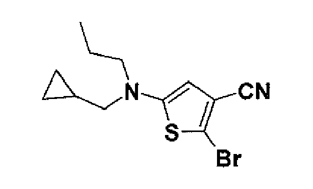

### (1) 2,5-dibromothiophene-3-carbaldehyde

3-thiophenecarboxyaldehyde (5.20 g, 46.4 mmol) and acetic acid (40 mL) were added, and bromine (5.3 mL, 2.2 eq) was dropped, then, the mixture was stirred at room temperature for 2 hours. Further, it was reacted at 65°C for 2 hours, then, cooled down to room temperature, and the resultant reaction mixture was poured into water, and extracted with t-butyl methyl ether. The organic layer was washed with saturated saline, dried over MgSO₄ then, concentrated under reduced pressure to obtain a residue, which was purified by silica gel column chromatography (eluent: hexane/ethyl acetate = 95/5) to obtain the title compound (7.33 g, 59%) as an oil.
¹H-NMR (300 MHz, CDCl₃): 7.32 (s, 1H), 9.77 (s, 1H)

### (2) 2,5-dibromothiophene-3-carbonitrile

2,5-dibromothiophene-3-carbaldehyde (1.03 g, 3.82 mmol) was dissolved in NMP (6 mL), hydroxylammonium sulfate (0.627 g, 1 eq) was added, and the mixture was heated at 100°C for 2 hours. After cooling, a NaHCO₃ aqueous solution was poured, the solution was extracted with t-butyl methyl ether, and the organic layer was washed with saturated saline, and dried over MgSO₄. Subsequently, the solvent was concentrated under reduced pressure to obtain a residue (1.09 g, 3.82 mmol), which was dissolved in toluene (30 mL), thionyl chloride (0.552 mL, 2 eq) was dropped, then, the mixture was heated at 100°C for 20 minutes. The reaction mixture was cooled down to room temperature, water was poured and the mixture was stirred for a while, then, NaHCO₃ was added to cause neutralization. After liquid-separation, the organic layer was washed with saturated saline, dried over MgSO₄, concentrated under reduced pressure to obtain a solvent, which was distilled off under reduced pressure to obtain the crude title compound (0.991 g, 97 %) as crystals.
¹H-NMR (300 MHz, CDCl₃): 7.11 (s, 1H)
GC/MS: m/z 265 (M), 267 (M+2), 269 (M+4)

### (3) 2-bromo-5-[(cyclopropylmethyl)(propyl)amino]thiophene-3-carbonitrile

2,5-dibromothiophene-3-carbonitrile (4) (0.991 g, 3.71 mmol), N-cyclopropylmethyl-N-propylamine (0.840 g, 2 eq), triethylamine (3.12 mL, 3 eq) and water (12 mL) were added, and the mixture was stirred at 100°C for 3 hours. Further, N-cyclopropylmethyl-N-propylamine (1.19 g, 2.8 eq) was additionally added, and the mixture was heated at 125°C for 30.5 hours. After cooling, the reaction mixture was poured into water, extracted with t-butyl methyl ether, and the organic layer was washed with saturated saline, dried over MgSO₄, then, distillation off under reduced pressure was performed to obtain a residue, which was purified by silica gel column chromatography (eluent: hexane/ethyl acetate = 9/1) to obtain the title compound (0.39 g, 35%) as an oil. ¹H-NMR (300 MHz, CDCl₃): δ 0.28-0.31 (m, 2H), 0.59-0.65 (m, 2H), 0.95 (t, J = 7.5 Hz, 3H), 1.09-1.14 (m, 1H), 1.69-1.76 (m, 2H), 3.32 (d, J = 6.6 Hz, 2H), 3.46-3.51 (m, 2H), 6.73 (s, 1H)
MS(ESI): m/z 299 (MH), 301 (MH+2)

### Example 7

### 5-[(cyclopropylmethyl)(propyl)amino]-2-(trifluoromethyl)thiophene-3-carbonitrile

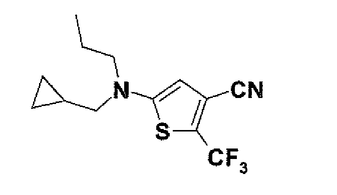

2-bromo-5-[(2-cyclopropylmethyl)(propyl)amino]thiophene-3-carbonitrile (0.366 g, 1.22 mmol), methyl fluorosulfonyldifluoroacetate (0.703 g, 3.0 eq), copper(I) iodide (50 mg, 20 mol%) and DMF (5.0 mL) were added, and the mixture was heated at 120°C for 3.5 hours. After cooling, the reaction mixture was poured into water, and liquid-separation thereof was performed using t-butyl methyl ether, the organic layer was washed with water, dried over MgSO₄, concentrated under reduced pressure to obtain a residue, which was purified by silica gel column chromatography (eluent: hexane/ethyl acetate = 95/5) to obtain the title compound (0.196 g, 55.7%) as an oil.
¹H-NMR (300 MHz, CDCl₃):δ 0.32-0.35 (m, 2H), 0.63-0.69 (m, 2H), 0.98 (t, J = 7.4 Hz, 3H), 1.11-1.18 (m, 1H) 1.71-1.83 (m, 2H), 3.39-3.42 (m, 2H), 3.55-3.61 (m, 2H), 7.16 (s, 1H) MS (ESI) :m/z 289 (MH)

### Example 8

### 3-bromo-5-(pyrrolidin-1-yl)thiophene-2-carbonitrile

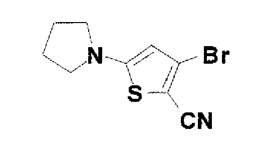

The title compound was obtained according to the methods in Example 1 and Example 2, using pyrrolidine instead of N-(cyclopropylmethyl)-N-propylamine.
¹H-NMR (300 MHz, CDCl₃): δ 2.09-2.11 (m, 4H), 3.27-3.32 (m, 4H), 5.67 (s, 1H)
MS(ESI): m/z 257 (MH), 259 (MH+2)

### Example 9

### 3-bromo-5-(2-methylpiperidin-1-yl)thiophene-2-carbonitrile

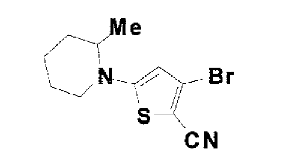

The title compound was obtained according to Example 1 and Example 2, using 2-methylpiperidine instead of N-(cyclopropylmethyl)-N-propylamine.
¹H-NMR (300 MHz, CDCl₃): δ 1.20 (d, J = 6.9 Hz, 3H), 1.58-1.82 (m, 6H), 3.03-3.12 (m, 1H), 3.27-3.33 (m, 1H), 3.84-3.88 (m, 1H), 5.86 (s, 1H)
MS(ESI): m/z 285 (MH), 287 (MH+2)

### Example 10

### 3-bromo-5-(piperidin-1-yl)thiophene-2-carbonitrile

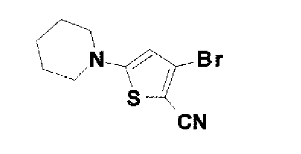

The title compound was obtained according to Example 1 and Example 2, using piperidine instead of N-(cyclopropylmethyl)-N-propylamine.
¹H-NMR (300 MHz, CDCl₃): δ 1.58-1.74 (m, 6H), 3.20-3.24 (m, 4H), 5.91 (s, 1H)
MS(ESI): m/z 271 (MH), 273 (MH+2)

### Example 11

### 3-bromo-5-(3,5-dimethylpiperidin-1-yl)thiophene-2-carbonitrile

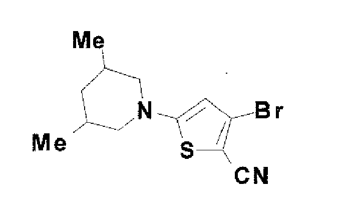

The title compound was obtained according to Example 1 and Example 2, using 3,5-dimethylpiperidine instead of N-(cyclopropylmethyl)-N-propylamine.
¹H-NMR (300 MHz, CDCl₃):δ 0.70-0.85 (m, 1H), 0.94 (s, 3H), 0.96 (s, 3H), 1.76-1.86 (m, 3H), 2.46 (t, J = 12.3 Hz, 2H), 3.41-3.46 (m, 2H), 5.92 (s, 1H)
MS(ESI):m/z 299 (MH), 301 (MH+2)

### Example 12

### 3-bromo-5-(4-methylpiperidin-1-yl)thiophene-2-carbonitrile

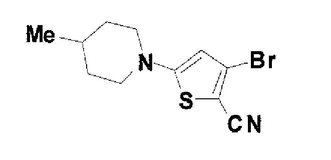

The title compound was obtained according to Example 1 and Example 2, using 4-methylpiperidine instead of N-(cyclopropylmethyl)-N-propylamine.
¹H-NMR (300 MHz, CDCl₃):δ 0.95 (d, J = 7.0 Hz, 3H), 1.30-1.77 (m, 5H), 2.90-2.99 (m, 2H), 3.48-3.54 (m, 2H), 5.91 (d, J = 4.8 Hz, 1H)
MS(ESI): m/z 285 (MH), 287 (MH+2)

### Example 13

### 3-bromo-5-[ (methyl)(2-methylpropyl)amino]thiophene-2-carbonitrile

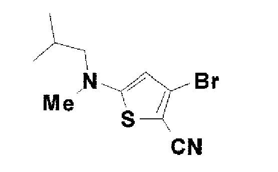

The title compound was obtained according to Example 1 and Example 2, using N-methyl-N-isobutylamine instead of N-(cyclopropylmethyl)-N-propylamine.
¹H-NMR (300 MHz, CDCl₃): δ 0.93 (s, 3H), 0.95 (s, 3H), 2.03-2.13 (m, 1H), 3.00 (s, 3H), 3.08 (d, J = 7.5 Hz, 2H), 5.73 (s, 1H)
MS(ESI): m/z 273 (MH), 275 (MH+2)

### Example 14

### 3-bromo-5- (dipropylamino)thiophene-2-carbonitrile

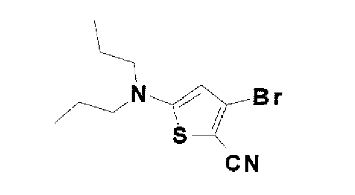

The title compound was obtained according to Example 1 and Example 2, using N,N-dipropylamine instead of N-(cyclopropylmethyl)-N-propylamine.
¹H-NMR (300 MHz, CDCl₃): δ 0.94 (t, J = 7.5 Hz, 6H), 1.56-1.72 (m, 4H), 3.21 (t, J = 7.5 Hz, 4H), 5.71 (s, 1H)
MS(ESI): m/z 287 (MH), 289 (MH+2)

### Example 15

### 3-bromo-5- (diallylamino)thiophene-2-carbonitrile

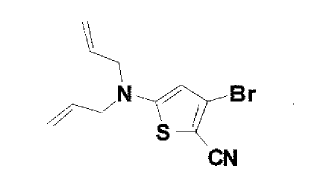

The title compound was obtained according to Example 1 and Example 2, using N,N-diallylamine instead of N-(cyclopropylmethyl)-N-propylamine.
¹H-NMR (300 MHz, CDCl₃):δ 3.88-3.91 (m, 4H), 5.20-5.31 (m, 4H), 5.71-5.84 (m, 3H)
MS(ESI):m/z 283 (MH), 285 (MH+2)

### Example 16

### 5-[bis(2-methoxyethyl)amino]-3-bromothiophene-2-carbonitrile

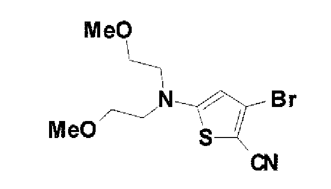

The title compound was obtained according to Example 1 and Example 2, using bis(2-methoxyethyl)amine instead of N-(cyclopropylmethyl)-N-propylamine.
¹H-NMR (300 MHz, CDCl₃): δ 3.35 (s, 6H), 3.35-3.60 (m, 8H), 5.82 (s, 1H)
MS(ESI): m/z 319 (MH), 321 (MH+2)

### Example 17

### 3-bromo-5-(2-ethylpiperidin-1-yl)thiophene-2-carbonitrile

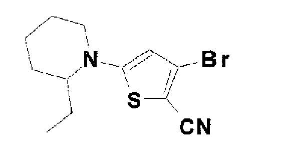

The title compound was obtained according to Example 1 and Example 2, using 2-ethylpiperidine instead of N-(cyclopropylmethyl)-N-propylamine.
¹H-NMR (300 MHz, CDCl₃): δ 0.95 (t, J = 7.5 Hz, 3H), 1.56-1.78 (m, 8H), 3.07-3.15 (m, 1H), 3.31-3.37 (m, 1H), 3.49-3.53 (m, 1H), 5.82 (s, 1H)
MS(ESI): m/z 299 (MH), 301 (MH+2)

### Example 18

### 3-bromo-5-(2-propylpiperidin-1-yl)thiophene-2-carbonitrile

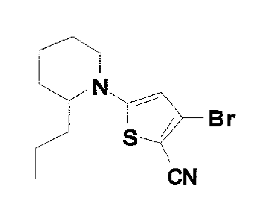

The title compound was obtained according to Example 1 and Example 2, using 2-propylpiperidine instead of N-(cyclopropylmethyl)-N-propylamine.
¹H-NMR (300 MHz, CDCl₃):δ 0.92-0.99 (m, 3H), 1.24-1.73 (m, 10H), 3.07-3.17 (m, 1H), 3.32-3.35 (m, 1H), 3.56-3.62 (m, 1H), 5.82 (s, 1H)
MS(ESI): m/z 313 (MH), 315 (MH+2)

### Example 19

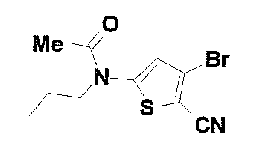

### (1) 3-bromo-5-(propylamino)thiophene-2-carbonitrile

3,5-dibromothiophene-2-carbonitrile (4.93 g, 18.3 mmol) and n-propylamine (12 mL) were added into a stainless autoclave, and the mixture was heated at 85 to 95°C for 6 hours. After cooling to room temperature, water was poured and the mixture was extracted with ethyl acetate, the organic layer was washed with saturated saline, and dried over MgSO₄. Subsequently, the solvent was distilled off under reduced pressure to obtain a residue, which was purified by silica gel column chromatography (eluent: hexane/ethyl acetate = 85/15), then, the resultant crystal was washed with a hexane-t-butyl methyl ether mixed solution (5/2, v/v) to obtain the title compound (1.78 g, 30%).
¹H-NMR (300 MHz, CDCl₃): 0.98-1.02 (m, 3H), 1.62-1.74 (m, 2H), 3.08-3.15 (m, 2H), 4.69 (brs, 1H), 5.89 (s, 1H)
MS(ESI): 245 (MH), 247 (MH+2)

### (2)N-(4-bromo-5-cyanothiophen-2-yl)-N-propylacetamide

3-bromo-5-(propylamino)thiophene-2-carbonitrile (0.173 g, 0.706 mmol), triethylamine (0.329 g, 4.6 eq) and anhydrous THF (3 mL) were added, and acetyl chloride (0.417 g, 7.5 eq) was dropped on ice. The mixture was stirred at room temperature for 18 hours, then, a saturated NaHCO₃ aqueous solution and ethyl acetate were added, and the mixture was stirred further for 30 minutes. Subsequently, the organic layer was separated, and washed with 5% hydrochloric acid and saturated saline sequentially, then, dried over MgSO₄. The solvent was distilled off under reduced pressure to obtain a crystalline residue, which was washed with t-butyl methyl ether to obtain the title compound (0.134 g, 66%).
¹H-NMR (300 MHz, CDCl₃): δ 1.00-1.07 (m, 3H), 1.77-1.84 (m, 2H), 2.41 (s, 3H), 3.81 (t, J = 7.8 Hz, 2H), 6.50 (s, 1H)
MS(ESI): m/z 287 (MH), 289 (MH+2)

### Example 20

### N-(4-bromo-5-cyanothiophen-2-yl)-N-propylpropanamide

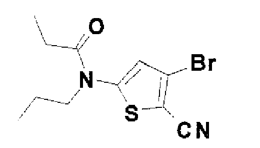

The title compound was obtained according to Example 19 using propanoyl chloride instead of acetyl chloride.
¹H-NMR (300 MHz, CDCl₃): δ 1.04 (t, J = 7.5 Hz, 3H), 1.26 (t, J = 7.2 Hz, 3H), 1.75-1.82 (m, 2H), 2.62 (m, 2H), 3.80 (t, J = 8.1 Hz, 2H), 6.50 (s, 1H)
MS(ESI): m/z 301 (MH), 303 (MH+2)

### Example 21

### N-(4-bromo-5-cyanothiophen-2-yl)-N-propylbutanamide

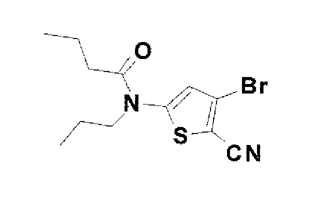

The title compound was obtained according to Example 19 using butanoyl chloride instead of acetyl chloride.
¹H-NMR (300 MHz, CDCl₃): δ 0.100-1.07 (m, 6H), 1.72-1.84 (m, 4H), 2.62 (m, 2H), 3.80 (t, J = 8.1 Hz, 2H), 6.50 (s, 1H)
MS(ESI): m/z 315 (MH), 317 (MH+2)

### Example 22

### N-(4-bromo-5-cyanothiophen-2-yl)-2-methyl-N-propylpropanamide

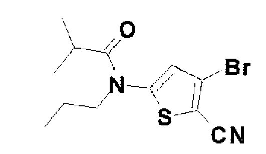

The title compound was obtained according to Example 19 using 2-methylpropanoyl chloride instead of acetyl chloride. ¹H-NMR (300 MHz, CDCl₃): δ 1.04 (t, J = 7.5 Hz, 3H), 1.24 (s, 3H), 1.26 (s, 3H), 1.76-1.84 (m, 2H), 2.59-3.10 (m, 1H), 3.84 (t, J = 7.8 Hz, 2H), 6.53 (s, 1H)
MS(ESI): m/z 315 (MH), 317 (MH+2)

### Example 23

### 3-bromo-5-[(propyl) (2,2,2-trifluoroethyl)amino]thiophene-2-carbonitrile

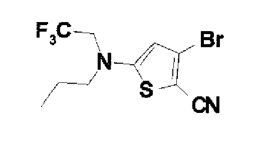

3-bromo-5-(propylamino)thiophene-2-carbonitrile (150 mg, 0.65 mmol) and TFA (5.0 mL) were added, and sodium borohydride (490 mg, 20 eq) was added on ice. After stirring at room temperature for 3 hours, the reaction mixture was concentrated under reduced pressure, a saturated NaHCO₃ aqueous solution was added, and the mixture was extracted with ethyl acetate. Subsequently, the organic layer was washed with saturated saline, then, dried over MgSO₄ The solvent was distilled off under reduced pressure to obtain a residue, which was purified by silica gel column chromatography (eluent: hexane/ethyl acetate = 10/1) to obtain the title compound (115 mg, 58%) as a colorless oil.
¹H-NMR (300 MHz, CDCl₃): δ 0.72-1.00 (m, 5H), 1.65-1.79 (m, 2H), 3.36 (t, J = 8.4 Hz, 2H), 4.12 (q, J = 7.3 Hz, 2H), 5.95 (s, 1H)
MS(ESI): m/z 327 (MH), 329 (MH+2)

### Example 24

### N-(4-bromo-5-cyanothiophen-2-yl)-N-propylglycine ethyl ester

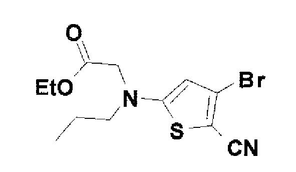

3-bromo-5-(propylamino)thiophene-2-carbonitrile (1.43 g, 5.83 mmol) was dissolved in anhydrous THF (56 mL), 60% NaH (0.280 g, 1.2 eq) was added on ice and the mixture was stirred for 30 minutes. Subsequently, ethyl bromoacetate (0.774 mL, 1.2 eq) was added, and the mixture was stirred for 2 hours while allowing its temperature to increase naturally up to room temperature. The reaction mixture was poured into water, extracted with ethyl acetate, the organic layer was washed with saturated saline, dried over MgSO₄ then, concentrated under reduced pressure to obtain the crude title compound (2.02 g, 100%) as crystals.
¹H-NMR (300 MHz, CDCl₃): δ 0.97 (t, J= 7.7 Hz, 3H), 1.23 (t, J = 3.7 Hz, 3H), 1.62-1.73 (m, 2H), 3.31 (t, J = 7.6 Hz, 2H), 3.99 (s, 2H), 4.23 (q, J = 7.3 Hz, 2H), 5.77 (s, 1H)
MS(ESI): m/z 331 (MH), 333 (MH+2)

### Example 25

### N2-(4-bromo-5-cyanothiophen-2-yl)-N-methyl-N2-propylglycinamide

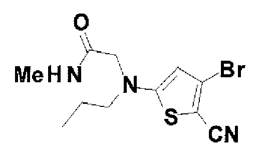

N-(4-bromo-5-cyanothiophen-2-yl)-N-propylglycine ethyl ester (100 mg, 0.30 mmol) was dissolved in ethanol (5.0 mL). Subsequently, a 40% methylamine aqueous solution (1 mL) was added, and the mixture was stirred at room temperature for 2 hours. The reaction mixture was concentrated under reduced pressure, then, the resultant residue was purified by silica gel column chromatography (eluent: hexane/ethyl acetate = 4/1) to obtain the title compound (70 mg, 74%) as white crystals. ¹H-NMR (300 MHz, CDCl₃): δ 0.97 (t, J = 7.3 Hz, 3H), 1.64-1.77 (m, 2H), 2.85 (d, J = 4.6 Hz, 3H), 3.31 (t, J = 7.8 Hz, 2H), 3.90 (s, 2H), 5.83 (s, 1H), 6.13 (br.s, 1H)
MS(ESI): m/z 316 (MH), 318 (MH+2)

### Example 26

### N-(4-bromo-5-cyanothiophen-2-yl)-N-propylglycine

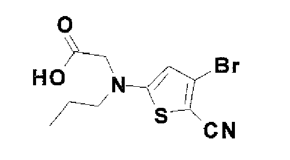

N-(4-bromo-5-cyanothiophen-2-yl)-N-propylglycine ethyl ester (2.02 g, 6.10 mmol) and methanol (6.5 mL) were added, 1N NaOH (6.5 mL, 1.1 eq) was dropped and the mixture was stirred at room temperature for 1 hour. The reaction mixture was poured into water, the aqueous layer was washed with t-butyl methyl ether, then, the aqueous layer was adjusted to acidic (pH = ca. 1) with hydrochloric acid, and extracted with ethyl acetate. The organic layer was washed with saturated saline, dried over MgSO₄, then, concentrated under reduced pressure to obtain the crude title compound (1.34 g, 72%) as a pale yellow solid.
¹H-NMR (300 MHz, CDCl₃): δ 0.97 (t, J = 7.4 Hz, 3H), 1.63-1.77 (m, 2H), 3.31 (t, J = 7.6 Hz, 2H), 4.1 (s, 2H), 5.79 (s, 1H)
MS(ESI): m/z 303 (MH), 305 (MH+2)

### Example 27

### N2-(4-bromo-5-cyanothiophen-2-yl)-N2-propylglycinamide

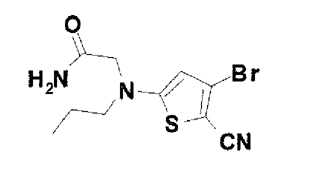

N-(4-bromo-5-cyanothiophen-2-yl)-N-propylglycine (0.716 g, 2.36 mmol) was suspended in toluene (14 mL), thionyl chloride (1.4 mL, 8.2 eq) was added and the mixture was heated at 80 to 85°C for 30 minutes. After cooling, the reaction mixture was concentrated under reduced pressure to obtain a residue, which was dissolved in toluene (4 mL), and 25% ammonia water (14 mL) was dropped while stirring vigorously on ice. The mixture was stirred at room temperature for 20 minutes, then, the reaction mixture was poured into water, and extracted with ethyl acetate. The organic layer was washed with saturated saline, dried over MgSO₄ then, concentrated under reduced pressure to obtain a residue, which was re-crystallized from t-butyl methyl ether to obtain the title compound (0.570 g, 80%) as pale yellow crystals.
¹H-NMR (300 MHz, CDCl₃): 5 0.98 (t, J = 7.5 Hz, 3H), 1.66-1.78 (m, 2H), 3.31-3.36 (m, 2H), 3.92 (s, 2H), 5.60 (br.s, 1H), 5.86 (s, 1H), 5.93 (br.s, 1H)
MS(ESI): m/z 302 (MH), 304 (MH+2)

### Example 28

### 3-bromo-5-[(cyanomethyl)(propyl)amino]thiophene-2-carbonitrile

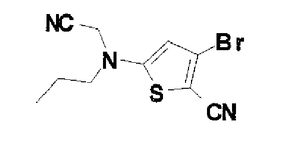

N2-(4-bromo-5-cyanothiophen-2-yl)-N2-propylglycinamide (0.270 g, 0.893 mmol) was dissolved in acetic anhydride (25 mL), and the mixture was heated at 135 to 140°C for 4 hours. After cooling, the reaction mixture was poured into water, and neutralized with Na₂CO₃. The aqueous layer was extracted with ethyl acetate, the organic layer was washed with saturated saline, dried over MgSO₄, then, the solvent was concentrated under reduced pressure to obtain a residue, which was purified by silica gel column chromatography (eluent: hexane/ethyl acetate = 65/35) to obtain the title compound (0.128 g, 50%) as pale yellow crystals.
¹H-NMR (300 MHz, CDCl₃): δ 1.00 (t, J = 7.5 Hz, 3H), 1.70-1.82 (m, 2H), 3.34 (t, J = 7.5 Hz, 2H), 4.16 (s, 2H), 6.02 (s, 1H)
MS(ESI): m/z 284 (MH), 286 (MH+2)

### Example 29

### N2-(4-bromo-5-cyanothiophen-2-yl)-N,N-dimethyl-N2-propylglycinamide

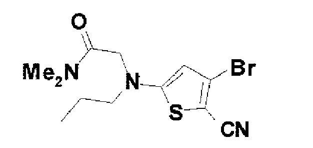

N-(4-bromo-5-cyanothiophen-2-yl)-N-propylglycine (0.300 g, 0.990 mmol) was suspended in toluene (6 mL), thionyl chloride (0.6 mL, 8.4 eq) was added, and the mixture was heated at 80 to 85°C for 30 minutes. After cooling, the reaction mixture was concentrated under reduced pressure to obtain a residue, which was dissolved in toluene (1 mL), and a 40% dimethylamine aqueous solution (4 mL) was added while stirring vigorously on ice. After stirring at room temperature for 30 minutes, the reaction mixture was poured into water, and extracted with ethyl acetate. The organic layer was washed with saturated saline, dried over MgSO₄, then, concentrated under reduced pressure to obtain a residue, which was re-crystallized from t-butyl methyl ether to obtain the title compound (0.279 g, 85%) as pale yellow crystals.
¹H-NMR (300 MHz, CDCl₃): δ 0.95 (t, J = 7.5 Hz, 3H), 1.62-1.72 (m, 2H), 2.99 (s, 3H), 3.04 (s, 3H), 3.31 (t, J = 7.5 Hz, 2H), 4.06 (s, 2H), 5.74 (s, 1H)
MS(ESI): m/z 330 (MH), 332 (MH+2)

### Example 30

### 3-bromo-5-[(1,3-dioxolan-2-ylmethyl)amino]thiophene-2-carbonitrile

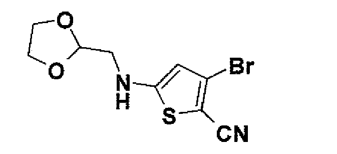

3,5-dibromothiophene-2-carbonitrile (3.22 g, 11.9 mmol), 2-(aminomethyl)-1,3-dioxolane (1.86 g, 1.5 eq), NaHCO₃ (2.95 g, 2.9 eq) and NMP (3.5 mL) were added, and the mixture was heated at 120°C for 4.5 hours. After cooling, the reaction mixture was poured into water, and the aqueous layer was extracted with ethyl acetate. The organic layer was washed with saturated saline, dried over MgSO₄, then, concentrated under reduced pressure to obtain a residue, which was purified by silica gel column chromatography (eluent: hexane/ethyl acetate = 7/3) to obtain the title compound (0.626 g, 19%) as orange crystals.
¹H-NMR (300 MHz, CDCl3): δ 3.36 (q, J = 3.3 Hz, 2H), 3.91-4.07 (m, 4H), 4.86 (br.s, 1H), 5.08 (t, J = 3.3 Hz, 1H), 5.97 (s, 1H)
MS(ESI): m/z 289 (MH), 291 (MH+2)

### Example 31

### 3-bromo-5-[(1,3-dioxolan-2-ylmethyl)(propyl)amino]thiophene-2-carbonitrile

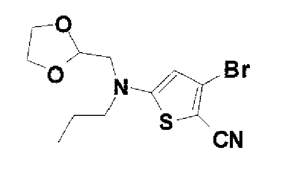

3-bromo-5-[(1,3-dioxolan-2-ylmethyl)amino]thiophene-2-carbonitrile (0.517 g, 1.79 mmol) was dissolved in DMF (10 mL), and 60% NaH (0.108 g, 1.5 eq) was added on ice, and the mixture was stirred at room temperature for 1 hour. Subsequently, propyl iodide (1.75 g, 5.8 eq) was dropped, the mixture was stirred at room temperature for 1 hour, then, the reaction mixture was poured into ice water, extracted with ethyl acetate, the organic layer was washed with saturated saline, and dried over MgSO₄. The solvent was concentrated under reduced pressure to obtain a residue, which was purified by silica gel column chromatography (eluent: hexane/ethyl acetate = 7/3) to obtain the title compound (0.515 g, 87%) as an oil.
¹H-NMR (300 MHz, CDCl₃): δ 0.94 (t, J = 7.5 Hz, 3H), 1.59-1.74 (m, 2H), 3.29-3.34 (m, 2H), 3.45 (d, J = 3.6 Hz, 2H), 3.86-4.03 (m, 4H), 5.04 (t, J = 3.6 Hz, 1H), 5.86 (s, 1H)
MS(ESI): m/z 331 (MH), 333 (MH+2)

### Example 32

### 3-bromo-5-[(2-oxoethyl)(propyl)amino]thiophene-2-carbonitrile

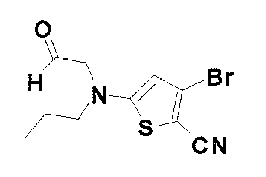

3-bromo-5-[(1,3-dioxolan-2-ylmethyl)(propyl)amino]thiophene-2-carbonitrile (0.443 g, 1.34 mmol) and methanol (20 mL) were added, 5N HCl (10 mL) was added, and the mixture was heated at 50 to 55°C for 1.5 hours. After the reaction mixture was cooled down to room temperature, it was neutralized with a 10% Na₂CO₃ aqueous solution, extracted with ethyl acetate, the organic layer was washed with saturated saline, and dried over MgSO₄ The solvent was concentrated under reduced pressure to obtain a residue, which was purified by silica gel column chromatography (eluent: hexane/ethyl acetate = 7/3) to obtain the title compound (0.220 g, 57%) as an oil.
¹H-NMR (300 MHz, CDCl₃): δ 0.97 (t, J = 7.5 Hz, 3H), 1.62-1.75 (m, 2H), 3.33 (t, J = 7.5 Hz, 2H), 4.07 (s, 2H), 5.73 (s, 1H), 9.67 (s, 1H)
MS(ESI): m/z 287 (MH), 289 (MH+2)

### Example 33

### 3-bromo-5-[propyl(3,3,3-trifluoro-2-hydroxypropyl)amino]thiophene-2-carbonitrile

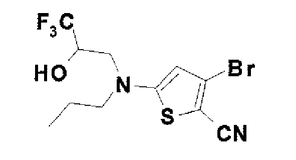

3-bromo-5-[(2-oxoethyl) (propyl)amino]thiophene-2-carbonitrile (0.0946 g, 0.329 mmol) was dissolved in anhydrous THF (10 mL), cesium fluoride (0.269 g, 5.4 eq) was added, and the mixture was cooled down to -78°C with dry ice/acetone. Subsequently, TMSCF₃ (80 µL, 1.6 eq) was added, and the mixture was stirred for 3 hours while allowing the reaction temperature to increase naturally up to room temperature. To the reaction mixture was added ethanol (2 mL), the mixture was stirred at room temperature for 30 minutes, water was poured and the mixture was extracted with ethyl acetate. The organic layer was washed with saturated saline, dried over MgSO₄, then, concentrated under reduced pressure to obtain a residue, which was purified by silica gel column chromatography (eluent: hexane/ethyl acetate = 8/2) to obtain the title compound (0.0869 g, 74%) as an oil.
¹H-NMR (300 MHz, CDCl₃): δ 0.93-1.06 (m, 3H), 1.62-1.77 (m, 2.6H), 3.09-3.65 (m, 4.4H), 4.36&4.73 (2brs, 1H), 5.80&5.89 (2s, 1H)
MS(ESI): m/z 357 (MH), 359 (MH+2)

### Example 34

### 3-bromo-5-[(2-hydroxypropyl)(propyl)amino]thiophene-2-carbonitrile

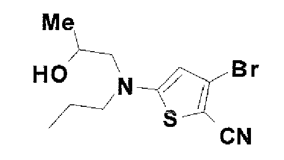

3-bromo-5-[(2-oxoethyl)(propyl)amino]thiophene-2-carbonitrile (0.0925 g, 0.322 mmol) was dissolved in anhydrous THF (4 mL), 3M MeMgBr (0.661 mL, 6.1 eq) was dropped on ice, then, the mixture was stirred at room temperature for 2 hours. Subsequently, to the reaction mixture was added an ammonium chloride aqueous solution, the mixture was extracted with ethyl acetate, and the organic layer was washed with saturated saline, and dried over MgSO₄ The solvent was concentrated under reduced pressure to obtain a residue, which was purified by silica gel column chromatography (eluent: hexane/ethyl acetate = 4/1) to obtain the title compound (0.0232 g, 24%) as an oil.
¹H-NMR (300 MHz, CDCl₃): δ 0.94 (t, J = 7.5 Hz, 3H), 1.24 (d, J = 6.3 Hz, 3H), 1.60-1.75 (m, 2H), 1.87 (d, J = 3.9 Hz, 1H), 3.19-3.32 (m, 4H), 4.13-4.18 (m, 1H), 5.79 (s, 1H)
MS(ESI): m/z 303 (MH), 305 (MH+2)

### Example 35

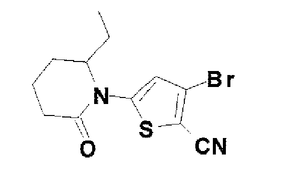

### (1) (±)-6-ethyl-2-piperidinone

Into a reaction vessel was added a 3.0M magnesium ethyl bromide/diethyl ether solution (50 mL, 150 mmol), it was cooled down to -78°C with dry ice/acetone, and a dichloromethane solution (200 mL) of glutarimide (5.0 g, 44 mmol) was dropped. The reaction temperature was raised gradually up to room temperature, then, the mixture was stirred further overnight at room temperature. To the reaction mixture was added sodium cyanoborohydride (3.32 g, 1.2 eq), then, 6N hydrochloric acid was added slowly so as to keep pH3 to 4. After stirring for 5 hours, the mixture was neutralized with a 10% sodium hydroxide solution to cause liquid-separation, and the aqueous layer was extracted with chloroform. The organic layer was washed with water, dried over Na₂SO₄, then, concentrated under reduced pressure to obtain a residue, which was purified by silica gel column chromatography (eluent: ethyl acetate/methanol = 9/1) to obtain the title compound (3.63 g, 65%) as colorless crystals.
¹H-NMR (300 MHz, CDCl₃): δ 0.94 (t, J = 7.5 Hz, 3H), 1.27-1.40 (m, 1H), 1.49-1.57 (m, 2H), 1.62-1.71 (m, 1H), 1.85-1.95 (m, 2H), 2.28-2.38 (m, 2H), 3.28-3.31 (m, 1H), 6.26 (brs, 1H)

### (2) 2-(3-bromo-5-iodothiophen-2-yl)-1,3-dioxolane

3,5-dibromothiophene-2-carbaldehyde (10.5 g, 38.9 mmol), ethylene glycol (22 mL, 10 eq), p-toluenesulfonic acid monohydrate (74 mg, catalytic amount) and toluene (150 mL) were added, and the mixture was refluxed with heating for 1 hour. After cooling, it was washed with a saturated NaHCO₃ aqueous solution and water sequentially, dried over Na₂SO₄, then, concentrated to obtain crude 2-(3,5-dibromothiophen-2-yl)-1,3-dioxolane (12.6 g) as an orange liquid.

The crude 2-(3,5-dibromothiophen-2-yl)-1,3-dioxolane (4.71 g, 15 mmol) and THF (150 mL) were added, the mixture was cooled down to -78°C with dry ice/acetone, and a 1.6M n-butyllithium/n-hexane solution (9.6 mL, 1.0 eq) was dropped. After 30 minutes, a THF solution (40 mL) of iodine (4.2 g, 1.1 eq) was dropped, the reaction temperature was raised gradually up to room temperature, then, the mixture was stirred further overnight at room temperature. To the reaction mixture was added a 5% sodium thiosulfate aqueous solution (100 mL), then, extracted with ethyl acetate. The organic layer was washed with water, dried over Na₂SO₄, then, concentrated under reduced pressure to obtain a residue, which was purified by silica gel column chromatography (eluent: hexane/ethyl acetate = 15/1) to obtain the title compound (3.68 g, 68%) as a yellow liquid.
¹H-NMR (300 MHz, CDCl₃): δ 4.02-4.15 (m, 4H), 6.08 (s, 1H), 7.10 (s, 1H)

### (3) 3-bromo-5-(2-ethyl-6-oxopiperidin-1-yl)thiophene-2-carbonitrile

6-ethyl-2-piperidinone (0.38 g, 2.99 mmol), copper iodide (22 mg, 5 mol%) and potassium phosphate (1.05 g, 2.0 eq) were added, and an atmosphere in the system was purged with nitrogen. Under nitrogen flow, 2-(3-bromo-5-iodothiophen-2-yl)-1,3-dioxolane (0.90 g, 2.49 mmol), N,N'-dimethylethylenediamine (28 µL, 10 mol%) and dioxane (3 mL) were added, and the mixture was stirred at 80°C for 28 hours. After allowing to cool, the reaction mixture was diluted with chloroform, filtrated through celite, then, concentrated under reduced pressure to obtain an orange liquid (1.30 g). This was purified by silica gel column chromatography (eluent: hexane/ethyl acetate = 2/1) to obtain an amide derivative (0.33 g) as a mixture with a regioisomer.

This crude amide derivative (0.33 g) was added to 80% acetic acid (12 mL), the mixture was stirred at room temperature for 1 hour, then, the reaction liquid was poured into a saturated NaHCO₃ aqueous solution (150 mL), extracted with ethyl acetate, and the organic layer was washed with water, dried over Na₂SO₄, then, concentrated to obtain an orange viscous material (0.30 g). This was stirred together with hydroxylamine sulfate (93 mg, 1.2 eq) in NMP (4 mL) at 110°C for 1 hour. After cooling, it was adjusted to alkaline (pH = ca. 9) with a 1N sodium hydroxide aqueous solution, extracted with ethyl acetate, washed with water, and dried. Subsequently, it was concentrated under reduced pressure to obtain a residue, and the residue and acetic anhydride (3 mL) were refluxed with heating for 3 hours. The reaction mixture was poured into ice water, stirred for a while, then, extracted with ethyl acetate. The organic layer was washed with water, dried over Na₂SO₄, then, concentrated under reduced pressure to obtain a residue, which was purified by silica gel column chromatography (eluent: hexane/ethyl acetate = 2/1) to obtain the title compound (0.23 g, 29%) as a pale yellow solid.
¹H-NMR (300 MHz, CDCl₃): δ 1.03 (t, J = 7.5 Hz, 3H), 1.66-1.75 (m, 1H), 1.85-2.14 (m, 5H), 2.64-2.73 (m, 2H), 4.03-4.08 (m, 1H), 6.48 (s, 1H)
GC-MS(EI): m/z 312 (M), 314 (M+2)

### Example 36

### 3-bromo-5-(2-ethyl-6-thioxopiperidin-1-yl)thiophene-2-carbonitrile

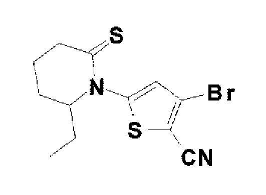

3-bromo-5-(2-ethyl-6-oxopiperidin-1-yl)thiophene-2-carbonitrile (0.29 g, 0.93 mmol), Lawesson's reagent (0.19 g, 0.5 eq) and dioxane (7 mL) were refluxed with heating for 1.5 hours. After allowing to cool, it was concentrated to obtain a residue, which was purified by silica gel column chromatography (eluent: hexane/ethyl acetate = 4/1) to obtain the title compound (0.22 g, 71%) as a yellow powder.
¹H-NMR (300 MHz, CDCl₃): δ 0.97-1.03 (m, 3H), 1.72-2.12 (m, 6H), 3.16-3.31 (m, 2H), 4.14-4.20 (m, 1H), 6.88 (s, 1H) GC-MS(EI): m/z 328 (M), 330 (M+2)

### Example 37

### N-(4-bromo-5-cyanothiophen-2-yl)-N-propylpropanthioamide

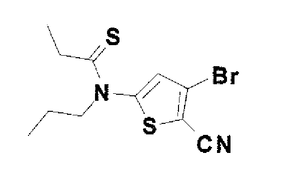

The title compound was obtained according to Example 36, using N-(4-bromo-5-cyanothiophen-2-yl)-N-propylpropanamide instead of 3-bromo-5-(2-ethyl-6-oxopiperidin-1-yl)thiophene-2-carbonitrile.
¹H-NMR (300 MHz, CDCl₃): δ 0.97 (t, J = 7.2 Hz, 3H), 1.30 (t, J = 6.9 Hz, 3H), 1.74-1.81 (m, 2H), 2.67 (brs, 2H), 4.15 (t, J = 8.1 Hz, 2H), 6.87 (s, 1H)
GC-MS(EI): m/z 316 (M), 318 (M+2)

### Example 38

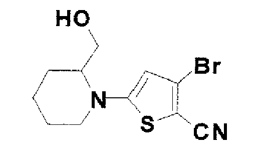

### (1) 2- (t-butyldimethylsilanyloxymethyl)-piperidine

2-piperidinemethanol (4.6 g, 40 mmol) and DMF (40 mL) were added, and imidazole (6.0 g, 2.2 eq) was added. Subsequently, t-butyldimethylsilyl chloride (6.6 g, 1.1 eq) was added, and the mixture was stirred at room temperature overnight. To the reaction mixture was added chloroform (100 mL), and it was washed with a saturated NaHCO₃ aqueous solution and water sequentially. After drying over Na₂SO₄, the mixture was concentrated under reduced pressure to obtain the crude title compound (8.48 g, 92%) as a yellow liquid. ¹H-NMR (300 MHz, CDCl₃): δ 0.05 (s, 6H), 0.89 (s, 9H), 1.02-1.14 (m, 1H), 1.26-1.62 (m, 4H), 1.76-1.81 (m, 1H), 2.56-2.67 (m, 2H), 3.06-3.11 (m, 1H), 3.38-3.43 (m, 1H),3.51-3.56 (m, 1H)

### (2) 3-bromo-5-[2- (tert-butyldimethylsilanyloxymethyl)-piperidin-1-yl]-thiophene-2-carbaldehyde

3,5-dibromothiophene-2-carbaldehyde (11.5 g, 42.7 mmol), 2- (t-butyldimethylsilanyloxymethyl)-piperidine (14.70 g, 1.5 eq), triethylamine (18 mL, 3.0 eq) and DMF (90 mL) were added, and the mixture was stirred at 110°C for 50 hours. After cooling, to the reaction mixture was added ethyl acetate (400 mL) and water (250 mL), liquid-separation thereof was performed, and the organic layer was washed with water, and dried over Na₂SO₄. It was concentrated under reduced pressure to obtain a residue, which was purified by silica gel column chromatography (eluent: hexane/ethyl acetate = 8/1) to obtain the title compound (7.27 g) as a regioisomer mixture.
¹H-NMR (300 MHz, CDCl₃): δ 0.01 (s, 6H), 0.82 (s, 9H), 1.61-1.91 (m, 6H), 3.10-3.19 (m, 1H), 3.46-3.50 (m, 1H), 3.72-3.75 (m, 2H), 3.81-3.87 (m, 1H), 6.02 (s, 1H), 9.56 (s, 1H)

### (3) 3-bromo-5-[2-(hydroxymethyl)piperidin-1-yl]thiophene-2-carbonitrile

The title compound was obtained according to Example 2, using 3-bromo-5-[2- (tert-butyldimethylsilanyloxymethyl)-piperidin-1-yl]-thiophene-2-carbaldehyde instead of 3-bromo-5-[(cyclopropyl)(propyl)amino]thiophene-2-carbaldehyde.
¹H-NMR (300 MHz, DMSO-d₆): δ 1.44-1.85 (m, 6H), 3.09-3.18 (m, 1H), 3.42-3.69 (m, 4H), 4.85 (t, J = 5.4 Hz, 1H), 6.28 (s, 1H) GC-MS(EI): m/z 300 (M), 301 (M+2)

### Example 39

### 3-bromo-5-(2-formylpiperidin-1-yl)thiophene-2-carbonitrile

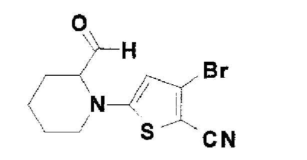

3-bromo-5-[2-(hydroxymethyl)piperidin-1-yl]thiophene-2-carbonitrile (0.39 g, 1.30 mmol), zinc oxide (0.53 g, 5.0 eq) and dichloromethane (80 mL) were cooled with ice water, a dichloromethane solution (8 mL) of N-t-butylbenzenesulfinimide chloride (0.42 g, 1.5 eq) was dropped, and the mixture was stirred for 2 hours. Water (10 mL) was added, then, the mixture was filtrated through celite, and the celite layer was washed with water and chloroform. The filtrate was separated, and the organic layer was washed with water, dried over Na₂SO₄, and concentrated to obtain a residue, which was purified by silica gel column chromatography (eluent: hexane/ethyl acetate = 2/1) to obtain the title compound (0.19 g, 50%) as a cream-colored powder.
¹H-NMR (300 MHz, CDCl₃): δ 1.26-1.31 (m, 1H), 1.62-1.95 (m, 4H), 2.32-2.37 (m, 1H), 3.26 (dt, J = 3.3 Hz, 12.3 Hz, 1H), 3.47-3.53 (m, 1H), 4.23 (d, J = 5.1 Hz, 1H), 5.91 (s, 1H), 9.66 (s, 1H)
MS(ESI): m/z 299 (MH), 301 (MH+2)

### Example 40

### 3-bromo-5-[2-(2,2,2-trifluoro-1-hydroxyethyl)piperidin-1-yl]thiophene-2-carbonitrile

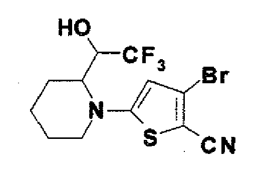

The title compound was obtained according to Example 33, using 3-bromo-5-(2-formylpiperidin-1-yl)thiophene-2-carbonitrile instead of 3-bromo-5-[(2-oxoethyl)(propyl)amino]thiophene-2-carbonitrile.
¹H-NMR (300 MHz, CDCl₃): 5 1.60-2.20 (m, 6H), 3.27-3.52 (m, 2H), 3.87-3.92 (m, 1H), 4.33-4.43 (m, 1H), 5.88&6.00 (2s, 1H)
MS(ESI): m/z 369 (MH), 371 (MH+2)

### Example 41

### 3-bromo-5-[2-(1-hydraxyethyl)piperidin-1-yl]thiophene-2-carbonitrile

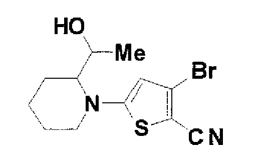

The title compound was obtained according to Example 34, using 3-bromo-5-(2-formylpiperidin-1-yl)thiophene-2-carbonitrile instead of 3-bromo-5-[(2-oxoethyl)(propyl)amino]thiophene-2-carbonitrile.
¹H-NMR (300 MHz, CDCl₃): δ 1.23&1.28 (2d, J = 6.3 Hz, 3H), 1.64-2.14 (m, 7H), 3.17-3.49 (m, 3H), 4.21-4.27 (m, 1H), 5.86&5.98 (2s, 1H)
MS(ESI): m/z 315 (MH), 317 (MH+2)

### Example 42

### 3-bromo-5-{[2-(hydroxyimino)ethyl](propyl)amino}thiophene-2-carbonitrile

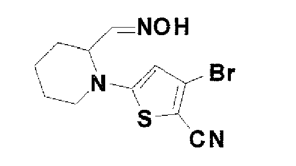

To an ethanol solution (2 mL) of 3-bromo-5-(2-formylpiperidin-1-yl)thiophene-2-carbonitrile (30 mg, 1.0 mmol) was added an aqueous solution (1 mL) of hydroxylamine sulfate (8 mg, 1.0 eq) and sodium acetate trihydrate (23 mg, 1.7 eq), and the mixture was refluxed with heating for 2 hours. After cooling, the reaction liquid was concentrated, to the residue was added ethyl acetate to effect extraction, and it was washed with water, dried and concentrated to obtain the title compound (25 mg, 80%) as a yellow brown solid.
¹H-NMR (300 MHz, CDCl₃): δ 1.59-2.11 (m, 6H), 3.22-3.27 (m, 1H), 3.38-3.49 (m, 1H), 4.35-4.39 (m, 0.65H), 4.90-4.94 (m, 0.35H), 5.95 (s, 1H), 6.80 (d, J = 6.3 Hz, 0.35H), 7.43 (d, J = 4.8 Hz, 0.65H)
MS(ESI): m/z 314 (MH), 316 (MH+2)

### Example 43

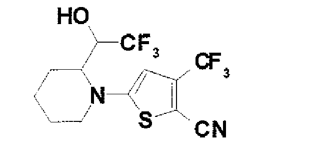

### (1) 5-[2-(acetoxymethyl)piperidin-1-yl]-3-bromothiophene-2-carbaldehyde

3-bromo-5-[2- (tert-butyldimethylsilanyloxymethyl)piperidin-1-yl]-thiophene-2-carbaldehyde (3.02 g, 7.22 mmol) and dioxane (18 mL) were added, and cooled with ice water, then, a 4M hydrogen chloride/1,4-dioxane solution (14 mL) was added, and the mixture was stirred for 1 hour. After neutralization with a saturated NaHCO₃ aqueous solution, it was extracted with ethyl acetate, and the organic layer was washed with water, dried over Na₂SO₄, then, concentrated under reduced pressure to obtain a residue, which was purified by silica gel column chromatography (eluent: hexane/ethyl acetate = 1/1) to obtain 3-bromo-5-[2-(hydroxymethyl)piperidin-1-yl]-thiophene-2-carbaldehyde (1.93 g, 6.34 mmol).

The total amount of the resultant compound and acetic anhydride (10 mL) were added into a reaction vessel, pyridine (0.54 mL) was added, then, the mixture was stirred at room temperature overnight. The reaction mixture was poured into ice water (100 mL), stirred for a while, then, extracted with ethyl acetate. The organic layer was washed with a saturated NaHCO₃ aqueous solution and water sequentially, and dried over Na₂SO₄. It was concentrated under reduced pressure to obtain a residue, which was purified by silica gel column chromatography (eluent: hexane/ethyl acetate = 2/1) to obtain the title compound (1.53 g, 73%) as an orange viscous material.
¹H-NMR (300 MHz, CDCl₃): 5 1.59-1.82 (m, 6H), 1.97 (s, 3H), 3.19-3.29 (m, 1H), 3.50-3.54 (m, 1H), 4.08-4.19 (m, 2H), 4.43-4.49 (m, 1H), 6.08 (s, 1H), 9.61 (s, 1H)

### (2) 5-[2-(2,2,2-trifluoro-1-hydroxyethyl)piperidin-1-yl]-3-trifluoromethyl-thiophene-2-carbonitrile

5-[2-(acetoxymethyl)piperidin-1-yl]-3-(trifluoromethyl)thiophene-2-carbaldehyde was obtained according to Example 4, using 5-[2-(acetoxymethyl)piperidin-1-yl]-3-bromothiophene-2-carbaldehyde instead of 3-bromo-5-[(cyclopropylmethyl)(propyl)amino]thiophene-2-carbaldehyde. Subsequently, the title compound was obtained by conducting the reactions according to Example 2, Example 39 and Example 33.
¹H-NMR (300 MHz, CDCl₃): 5 1.64-1.90 (m, 6H), 2.85 (brs, 1H), 3.26-3.53 (m, 2H), 3.92-3.98 (m, 1H), 4.35-4.44 (m, 1H), 6.06&6.16 (2s, 1H)
GC-MS(EI): m/z 358 (M)

### Example 44

### Optical resolution of 3-bromo-5-[2-(2,2,2-trifluoro-1-hydroxyethyl)piperidin-1-yl]thiophene-2-carbonitrile

The racemate 3-bromo-5-[2-(2,2,2-trifluoro-1-hydroxyethyl)piperidin-1-yl]thiophene-2-carbonitrile obtained according to Example 40 was subjected to diastereomer separation in a ODS column. Subsequently, enantiomers were fractionated by a chiral column, to obtain four kinds of optical isomers.

3-bromo-5-[2-(2,2,2-trifluoro-1-hydroxyethyl)piperidin-1-yl]thiophene-2-carbonitrile (77.1 mg, 0.209 mmol) was dissolved in acetonitrile (10 mL) and the solution was subjected to preparative HPLC (column: YMC ODS-AM 2 cmx25 cm, elution solvent: water/acetonitrile = 1/1 v/v, flow rate: 15 mL/min, injection volume: 500 to 800 µL) to obtain diastereomer isomers [component A (t_{R} = 25.7 min): 44.6 mg, 57.8%; component B (t_{R} = 26.9 min): 24.1 mg, 31.3%)].

Subsequently, optical isomers of the components A and B were subjected to preparative HPLC (column: CHIRALPAK IA 2 cmx25 cm, elution solvent: hexane/IPA = 95/5 v/v, flow rate: 15 mL/min) using a chiral column, to obtain four kinds of optical isomers: a component A-1 (t_{R} = 13.5 min) and a component A-2 (t_{R} = 14.9 min) from the component A and a component B-1 (t_{R} = 15.2 min) and a component B-2 (t_{R} = 22.5 min) from the component B.
Component A-1: crystal (20.5 mg, 26.6%, m.p. 122-123°C)
Component A-2: crystal (20.7 mg, 26.8%, m.p. 104-106°C)
Component B-1: amorphous (9.60 mg, 12.5%)
Component B-2: amorphous (9.20 mg, 11.9%)

### Diastereomer (component A)

¹H-NMR (300 MHz, CD₃OD): δ 1.45-1.78 (m, 5H), 2.06 (d, 1H, J = 13.2 Hz), 3.20-3.45 (m, 3H), 3.75-3.78 (m, 1H), 4.31-4.41 (m, 1H), 5.99 (s, 1H)
MS(ESI): m/z 369 (MH), 371 (MH+2)

### Diastereomer (component B)

¹H-NMR (300 MHz, CD₃OD): δ 1.52-1.76 (m, 6H), 3.20-3.29 (m, 2H), 3.41-3.47 (m, 1H), 3.82-3.86 (m, 1H), 4.31-4.41 (m, 1H), 5.99 (s, 1H)
MS(ESI): m/z 369 (MH), 371 (MH+2)

### Example 45

### 5-[(propyl)(2,2,2-trifluoroethyl)amino]-3-trifluoromethylthiophene-2-carbonitrile

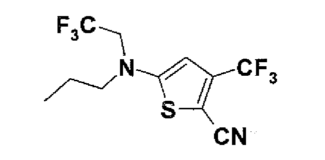

3-bromo-5-[(propyl)(2,2,2-trifluoroethyl)amino]thiophene-2-carbonitrile (50 mg, 0.15 mmol) obtained according to Example 23 and DMF (2 mL) were charged into a reaction vessel, methyl fluorosulfonyldifluoroacetate (147 mg, 5.0 eq) and copper iodide (29 mg, 1.0 eq) were added, and the mixture was heated at 110 to 120°C for 5 hours. After cooling, to the reaction mixture was added water, it was extracted with ethyl acetate, and the organic layer was washed with saturated saline, then, dried over Na₂SO₄. The solvent was concentrated under reduced pressure to obtain a residue, which was purified by silica gel column chromatography (eluent: hexane/ethyl acetate = 94/6) to obtain the title compound (28 mg, 58%) as a pale yellow oil.
¹H-NMR (270 MHz, CDCl₃): δ 0.98 (t, J = 8.1 Hz, 3H), 1.67-1.81 (m, 2H), 3.38 (t, J = 8.1 Hz, 2H), 3.82-3.92 (m, 2H), 6.11 (s, 1H)
GC-MS(EI): m/z 302 (M)

### Example 46

### 5-[(propyl)(2,2,2-trifluoroethyl)amino]-3-methylthiophene-2-carbonitrile

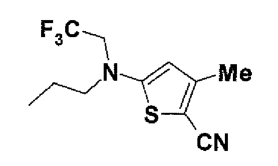

3-bromo-5-[(propyl)(2,2,2-trifluoroethyl)amino]thiophene-2-carbonitrile (82 mg, 0.25 mmol) obtained according to Example 23 and DMA (1.5 mL) were charged into a reaction vessel, Me₄Sn (90 mg, 2.0 eq) and PdCl₂(PPh₃)₂ (9 mg, 5 mol%) were added under nitrogen atmosphere, and the mixture was stirred at 75 to 80°C for 7 hours. After cooling, to the reaction mixture was added a saturated NH₄Cl aqueous solution, it was extracted with ethyl acetate, and the organic layer was washed with saturated saline, then, dried over MgSO₄. The solvent was concentrated under reduced pressure to obtain a residue, which was purified by silica gel column chromatography (eluent: hexane/ethyl acetate = 10/1) to obtain the title compound (30 mg, 46%) as a colorless oil.
¹H-NMR (270 MHz, CDCl₃): δ 0.95 (t, J = 7.4 Hz, 3H), 1.61-1.80 (m, 2H), 2.30 (s, 3H), 3.32 (t, J = 7.8 Hz, 2H), 3.82 (q, J = 8.6 Hz, 2H), 5.80 (s, 1H)
GC-MS(EI): m/z 263 (M)

### Example 47

### 5-(2-cyanopiperidin-1-yl)-3-bromo-thiophene-2-carbonitrile

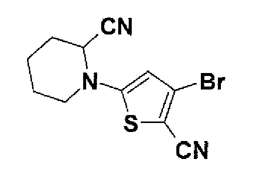

3-bromo-5-(2-formylpiperidin-1-yl)thiophene-2-carbonitrile (90 mg, 0.30 mmol) obtained according to Example 39 and NMP (5 mL) were charged into a reaction vessel, hydroxylammonium sulfate (60 mg, 1.2 eq) was added, and the mixture was stirred at room temperature for 1 hour, then, acetic anhydride (0.1 mL, 3.6 mmol) was added and the mixture was stirred at 100°C for 1 hour. After cooling, to the reaction mixture was added a saturated NaHCO₃ aqueous solution, it was extracted with ethyl acetate, and the organic layer was washed with saturated saline, then, dried over MgSO₄. The solvent was concentrated under reduced pressure to obtain a residue, which was purified by silica gel column chromatography (eluent: hexane/ethyl acetate = 2/1) to obtain the title compound (64 mg, 72%) as a white powder.
¹H-NMR (270 MHz, CDCl₃): δ 1.26-2.36 (m, 6H), 3.09-3.15 (m, 1H), 3.41-3.47 (m, 1H), 4.53(br.s, 1H), 6.16 (s, 1H)
MS(ESI): m/z 296 (MH), 298 (MH+2)

The activity of the inventive compound via an androgen receptor was measured using the following in vitro assay.

### Reference Example 1

### Preparation of plasmid containing responsive reporter gene

An oligonucleotide (5'-CTAGTAAAGTACTCCAAGAACCTATTTGAGCT-3') of SEQ ID NO: 6 comprising a sequence in which a Sac I linker sequence is linked to an ARE sequence of rat probasin gene promoter, and an oligonucleotide (5'-CAAATAGGTTCTTGGAGTACTTTACTAGAGCT-3') of SEQ ID NO: 7 comprising a sequence complementary to the above oligonucleotide were synthesized, respectively, and the end of the respective oligonucleotides was phosphorylated by T4 polynucleotide kinase. These phosphorylated oligonucleotides were mixed and kept at 80°C for 10 minutes, then, cooled gradually to fabricate a double-stranded DNA. The resultant DNA was purified with phenol/chloroform/isoamylalcohol (PCI), then, a part of this was subjected to a ligation reaction using a ligation kit (manufactured by Takara Shuzo Co., Ltd.), and the reaction product was subjected to electrophoresis using 3% Nusive GTG agarose. DNA was recovered from a gel of a band portion of the reaction product having a sequence containing 2 to 4 repetitions of the above-described oligonucleotide sequence, based on the mobility of a molecular weight marker. The resultant DNA is termed (2-4)×ARE DNA.

Two oligonucleotides (5'-GATCTCGACTATAAAGAGGGCAGGCTGTCCTCTAAGCGTCACCACGACTTCA-3' and 5'-AGCTTGAAGTCGTGGTGACGCTTAGAGGACAGCCTGCCCTCTTTATAGTCGA-3') of SEQ ID NOs. 8 and 9 comprising nucleotide sequences derived from a nucleotide sequence around TATA box and a leader sequence of mouse metallothionein I gene (Genbank Accession No. J00605) were annealed to obtain a double-stranded DNA, and T4 polynucleotide kinase was allowed to act on this to phosphorylate both the ends (hereinafter, the DNA is referred to as TATA DNA). Meanwhile, plasmid pGL3 (manufactured by Promega K.K.) containing firefly luciferase gene was digested with restriction enzymes Bgl II and Hind III, then, to this was added Bacterial alkaline phosphatase (BAP) and the mixture was kept at 65°C for 1 hour. Subsequently, the resultant mixture was subjected to electrophoresis using low-melting point agarose (Agarose L; manufactured by Nippon Gene Co., Ltd.), to recover DNA of Bgl II-Hind III fragment containing pGL3-derived luciferase gene. About 100 ng of the DNA and 1 µg of the above-described TATA DNA were mixed, and linked by T4 ligase to prepare plasmid pGL3-TATA.

Next, pGL3-TATA was digested with a restriction enzyme Sac I, then, BAP was added thereto and the mixture was kept at 65°C for 1 hour. The resultant mixture was subjected to electrophoresis with low-melting point agarose gel, and DNA was recovered from a gel of a band portion. About 100 ng of the DNA and about 1 µg of the above-described (2-4)×ARE DNA were mixed and T4 ligase was reacted, then, and DNA in the reaction liquid was introduced into DH5α competent cell (manufactured by TOYOBO). From several colonies of E. coli showing ampicillin resistance, DNAs of plasmids contained in them were prepared, and these were digested with restriction enzymes Kpn I and Bgl II and the digested liquid was analyzed by agarose gel electrophoresis. The plasmid having a structure containing 3 copies of ARE DNA introduced into Sac I site of pGL3-TATA was named pGL3-TATA-3×ARE.

### Reference Example 2

### Preparation of androgen receptor expression plasmid

For obtaining cDNA having a nucleotide sequence encoding an amino acid sequence of human-derived wild androgen receptor, a forward primer comprising the nucleotide sequence of SEQ ID NO: 1 and a reverse primer comprising the nucleotide sequence of SEQ ID NO: 2 were synthesized using a DNA synthesizer (model 394, manufactured by Applied Biosystems).

Human Prostate cDNA (10 ng)(QUICK-Clone cDNA no. 7123-1 manufactured by Clontech) as a template, each 10 pmol of the above-described primers, LA-Taq polymerase (manufactured by Takara Shuzo Co., Ltd.) and a buffer appended to the enzyme were used to prepare the reaction liquid of 50 µ1, then, PCR was performed. In this PCR, PCRsystem9700 (manufactured by Applied Biosystems) was used, and was conducted 35 cycles of incubation each cycle including incubation at 95°C for 1 minute followed by incubation at 68°C for 3 minutes.

After conducting PCR, the total amount of the reaction liquid was subjected to agarose gel electrophoresis using low-melting point agarose (Agarose L: manufactured by Nippon Gene Co., Ltd.). It was observed by ethidium bromide staining that DNA of size expected from a known nucleotide sequence was amplified, then, the DNA was recovered. A sample for direct sequencing was prepared using a part of the recovered DNA and Dye Terminator Sequence Kit FS (manufactured by Applied Biosystems), and this was subjected to nucleotide sequence analysis using an autosequencer (manufactured by Applied Biosystems, model 3700). As a result, it was observed that the recovered DNA had a nucleotide sequence encoding the amino acid sequence of SEQ ID NO: 3.

PCR was carried out using about 100 ng of DNA recovered as described above as a template, a forward primer comprising the nucleotide sequence of SEQ ID NO: 4 and a reverse primer comprising the nucleotide sequence of SEQ ID NO: 5, to amplify DNA having a structure containing a nucleotide sequence encoding an amino acid sequence of human standard androgen receptor linked immediately after Kozak consensus sequence. That is, 0.1 µg of the DNA as a template, each 10 pmol of the above-described primers, LA-Taq polymerase (manufactured by Takara Shuzo Co., Ltd.) and a reaction buffer appended to the enzyme were used to prepare the reaction liquid of 50 µ1, then, PCR was carried out. In this PCR, PCRsystem9700 (manufactured by Applied Biosystems) was used, and was conducted 20 cycles of incubation each cycle including incubation at 95°C for 1 minute followed by incubation at 68°C for 3 minutes. After conducting PCR, the total amount of the reaction liquid was subjected to electrophoresis with low-melting point agarose gel to separate DNA and DNA was recovered. About 1 µg of the recovered DNA was treated by DNA blunting kit (manufactured by Takara Shuzo Co., Ltd.) to blunt the ends thereof, then, was reacted with T4 polynucleotide kinase to phosphorylate its ends. The resultant DNA was treated with phenol, then, further purified by ethanol precipitation. The total amount of the purified DNA was used as insert DNA for preparing expression plasmid described later.

Plasmid pRc/RSV (Manufactured by Invitrogen) was digested with a restriction enzyme Hind III, then, to the digested material was added BAP and the mixture was kept at 65°C for 1 hour. The resultant mixture was treated with phenol, then, further purified by ethanol precipitation. The purified DNA was treated by Blunting kit (manufactured by Takara Shuzo Co., Ltd.) to blunt its ends, then, this was subjected to electrophoresis using low-melting point agarose (manufactured by Nippon Gene Co., Ltd.; agarose L), and DNA was recovered from a gel at a band portion. About 100 ng of the recovered DNA and the total amount of the above-described insert DNA were mixed, and T4 ligase was added to this and reacted. The resultant reaction liquid was used to transform E. coli DH5α competent cells. From several colonies of E. coli showing ampicillin resistance, DNAs of plasmids contained in them were prepared, and the nucleotide sequence of the prepared DNA was determined by a dye terminator method using ABI model 3700 type autosequencer. A plasmid was selected in which perfect matching of nucleotide sequences in translation region was observed by comparing the determined nucleotide sequence and the nucleotide sequence obtained by the above-described direct sequence, and this was named pRc/RSV-hAR Kozak.

### Test Example 1

### Measurement of androgen receptor agonist activity of test substance by reporter gene assay method

The compound having an androgen agonist activity was identified by a reporter gene assay method using cells for measuring an ability of modulating androgen receptor activity manifestation.

Using a medium prepared by adding charcoal/dextrantreated FBS to DMEM medium (manufactured by Nacalai Tesque Inc. or Wako Pure Chemical Industries, Ltd.) so that the final concentration of the FBS will be 10%, transformed cells were inoculated at a rate of about 1×10⁵ cells/well in a 96-well plate for luciferase light emission measurement and for culture (#3917 manufactured by Corning Costar Corporation), which transformed cells had been prepared by stably introducing into human-derived CHP212 cells DNA of plasmid pGL3-TATA-3×ARE comprising a nucleotide sequence in which firefly luciferase gene was linked to a downstream of a 3×ARE sequence containing 3 repetitions of the ARE sequence of rat probasin gene promoter and TATA box sequence was contained at upstream of the 3×ARE sequence (see, Reference Example 1) and DNA of plasmid pRC/RSV-hAR Kozak (see, Reference Example 2), then, DHT (namely, androgen receptor agonist) dissolved in DMSO was added to the transformed cells. The solutions were prepared and added so that the final concentration of DHT will increase each by 10-fold for every test wells and the final DMSO amounts in cultures in all test wells will be the same (0.1%). As a group containing no added DHT, was prepared a control well to which only a solvent (DMSO) was added.

These cells were cultured, and 24 hours after addition of DHT, the medium was removed, and 50 µL of a luciferase emission reagent Steady-Glo was added thereto. After shaking for 10 minutes, the luciferase activity was measured by Envision 2103 Multilabel Reader (PerkinElmer Co., Ltd.). In the cells for measuring an ability of modulating an androgen receptor activity, an increase was observed in the luciferase activity associated with an increase in the concentration of DHT added to the cells.

Instead of DHT, a test substance was added to each test well and examined, in the same manner as described above, thereby carrying out measurement of the agonist activity of the test substance on an androgen receptor. The results are shown in Table 1.

**Table 1**

| Example No. of test substance | EC50 |
|---|---|
| 1 | >10 µM |
| 2 | 10.6 nM |
| 5 | 11.9 nM |
| 7 | 99.3 nM |
| 8 | 1.4 µM |
| 9 | 0.45 µM |
| 12 | 1.5 µM |
| 13 | 1.2 µM |
| 14 | 43.3 nM |
| 15 | 13.1 nM |
| 16 | 1.6 µM |
| 17 | 4.9 µM |
| 18 | 36.2 µM |
| 23 | 5.4 µM |
| 24 | >10 µM |
| 25 | 1.5 µM |
| 26 | >10 µM |
| 28 | 0.58 µM |
| 31 | 0.75 µM |
| 32 | >10 µM |
| 35 | >10 µM |
| 40 | 14.0 nM |
| 41 | 0.56 µM |
| 43 | >10 µM |
| 45 | 3.1 nM |
| 46 | 25.2 nM |
| 47 | 1.5 µM |

Table 1 teaches that the inventive compounds show excellent agonist activity on an androgen receptor.

### Test Example 2

### Test of S9 metabolism stability in human, rat and mouse for inventive compound

For the inventive compound, was carried out test of a S9 metabolism stability in human, rat and mouse.

### Preparation of calibration curve sample and internal standard (I.S.; reserpine)-added blank sample

Using MINISORP(75x12) NUNC-IMMUNO tube, a 0.5 µM I.S.-added reaction stopping solution (500 µL) and a 10 mmol/L NADPH solution (50 µL) were mixed. Subsequently, a S9 mixture (445 µL) and pure water (5 µL) were added to prepare an I.S.-added blank sample. Likewise, a S9 mixture (445 µL) and a 100 µM test substance solution (5 µL) were added, preparing an calibration curve sample.

### Preparation of sample for measuring metabolism stability

A S9 mixture (1068 µL) was dispensed into MINISORP (75x12) NUNC-IMMUNO tube. On ice water, a 100 µM test substance solution (12 µL) was added, and pre-incubation was carried out at 37°C for 5 minutes. An aliquot (450 µL) of this was fractionated, and a 0.5M I.S.-added reaction stopping solution (500 µL) and a 10 mmol/L NADPH solution (50 µL) were added, to obtain an unreacted sample (reaction time; 0 min). To the residue after fractionation, a 10 mmol/L NADPH solution (70 µL) was added, and incubation was carried out at 37°C for 30 minutes, and an aliquot (500 µL) was fractionated, then, a 0.5 µM I.S.-added reaction stopping solution (500 µL) was added, preparing a reacted sample (reaction time; 30 min).

### Pre-treatment

Each sample was centrifuged (2700 rpm, 10 min, r.t.), then, the supernatant (150 µL) was diluted in a 96-well deep well plate (1.1 mL internal volume) into which 150 µL of water/methanol (= 1/1(v/v)) had been dispensed previously. Subsequently, 96-well Nunc plate (0.5 mL inner volume) was attached to a lower part of MultiScreen Solvinert 96-well plate (hydrophilic), and the supernatant (150 µL) of the sample was added. After centrifugation (1900 rpm, 2 min, r.t.), LC/MS/MS analyses were carried out under the following HPLC operation conditions.

### HPLC operation conditions

### High Performance Liquid Chromatography System: Ultra Performance LC System (Waters Corp.)

Analysis Column: ACQUITY UPLC BEH C18 50 mm L. × 2.1 mm I.D., 1.7 µm (Waters Corp.)

| | |
|---|---|
| Column temperature : | 40°C |
| Mobile phase : | A; H₂O/MeCN/AcOH = 90/10/1 (v/v/v) |
| | B; H₂O/MeCN/AcOH = 20/80/1 (v/v/v) |

| Gradient | | | | | | |
|---|---|---|---|---|---|---|
| Time (min) | 0.00 | 0.30 | 0.30 | 1.50 | 1.50 | 3.00 |
| Liquid B(%) | 0.0 | 0.0 | 100.0 | 100.0 | 0.0 | 0.0 |

| | |
|---|---|
| Flow Rate | : 0.30 mL/min |
| Run Time | : 3 min |
| Autosampler temperature | : set temperature 4°C |

The measurement results of the test of S9 metabolism stability in human, rat and mouse are described as residual ratio (%) in Table 2.

**Table 2**

| Structural formula (Example No.) | Residual ratio (%) | | |
|---|---|---|---|
| | human | rat | mouse |
| 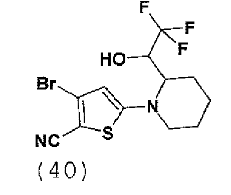 | 73.4 | 40.1 | 39.1 |

Table 2 teaches that the inventive compound shows excellent metabolism stability. Particularly, the metabolism stability in human is excellent.

### INDUSTRIAL APPLICABILITY

The inventive compound is useful for treatment or prevention of conditions or disorders having sensitivity to selective androgen receptor modulation, further, excellent in metabolism stability, thus, it can be provided as a useful androgen receptor agonist and a pharmaceutical composition containing them as an active ingredient.

### FREE TEXT IN SEQUENCE LISTING

SEQ ID NO:1
   Designed oligonucleotide primer for PCR
SEQ ID NO:2
   Designed oligonucleotide primer for PCR
SEQ ID NO:4
   Designed oligonucleotide primer for PCR
SEQ ID NO:5
   Designed oligonucleotide primer for PCR
SEQ ID NO:6
   Designed oligonucleotide
SEQ ID NO:7
   Designed oligonucleotide
SEQ ID NO:8
   Designed oligonucleotide
SEQ ID NO:9
   Designed oligonucleotide

## Claims

1. A compound represented by the formula (I): 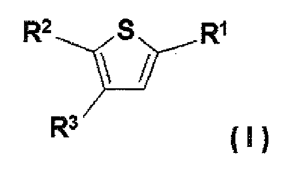 [wherein:
R¹ is a group represented by the formula (II): 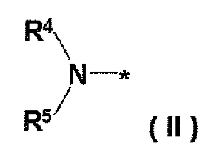
{wherein:
R⁴ represents -(Q¹)x-R⁶;
Q¹ represents alkylene;
X is 0 or 1;
R⁶ represents hydrogen, alkyl, cycloalkyl, alkenyl, alkynyl, halogen, haloalkyl or alkoxy;
R⁵ represents -(Q²)y¹-(Q³)y²-R⁷;
Q² represents alkylene;
Q³ represents -C(O)- or C (s)-;
y¹ is 0 or 1;
y² is 0 or 1;
R⁷ represents hydrogen, alkyl, cycloalkyl, alkenyl, alkynyl, halogen, haloalkyl, hydroxy, alkoxy, cyano, - N(R⁸)(R⁹) or -CH(R¹⁰)(R¹¹);
R⁸ and R⁹ represent each independently hydrogen, alkyl, cycloalkyl, alkenyl or alkynyl;
R¹⁰ and R¹¹ represent each independently alkyl, cycloalkyl, alkenyl, alkynyl, halogen, haloalkyl, hydroxy, alkoxy or cyano, alternatively R¹⁰ and R¹¹ together form alkylenedioxy (if either R¹⁰ or R¹¹ is hydroxy, another is a moiety other than hydroxy and alkoxy)}
or a group represented by the formula (III): 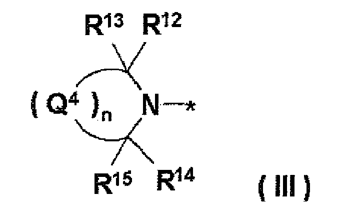 {wherein:
Q⁴ represents optionally substituted methylene;
n is 1 to 5;
R¹² and R¹³ represent each independently -(Q⁵)z-R¹⁶, alternatively form = O or = S together with a carbon atom to which they are bonded;
Q⁵ represents alkylene;
z is 0 or 1;
R¹⁶ represents hydrogen, alkyl, cycloalkyl, alkenyl, alkynyl, halogen, haloalkyl, hydroxy, alkoxy, cyano, formyl, - CH=N-R¹⁷, -N(R¹⁸)(R¹⁹) or -CH(R²⁰)(R²¹)_{;}
R¹⁷ represents hydrogen, alkyl, cycloalkyl, alkenyl, alkynyl or hydroxy;
R¹⁸ and R¹⁹ represent each independently hydrogen, alkyl, cycloalkyl, alkenyl or alkynyl;
R²⁰ and R²¹ represent each independently alkyl, cycloalkyl, alkenyl, alkynyl, halogen, haloalkyl, hydroxy, alkoxy or cyano (if either R²⁰ or R²¹ is hydroxy, another is a moiety other than hydroxy and alkoxy);
R¹⁴ and R¹⁵ represent each independently -(Q⁶)w-R²²;
Q⁶ represents alkylene;
w is 0 or 1;
R²² represents hydrogen, alkyl, cycloalkyl, alkenyl, alkynyl, halogen, haloalkyl, hydroxy, alkoxy, cyano, formyl, - CH=N-R²³, -N(R²⁴)(R²⁵) or -CH(R²⁶)(R²⁷);
R²³ represents hydrogen, alkyl, cycloalkyl, alkenyl, alkynyl or hydroxy;
R²⁴ and R²⁵ represent each independently hydrogen, alkyl, cycloalkyl, alkenyl or alkynyl;
R²⁶ and R²⁷ represent each independently alkyl, cycloalkyl, alkenyl, alkynyl, halogen, haloalkyl, hydroxy, alkoxy or cyano (if either R²⁶ or R²⁷ is hydroxy, another is a moiety other than hydroxy and alkoxy)};
R² represents cyano, formyl, nitro, halogen or haloalkyl;
R³ represents cyano, nitro, halogen, haloalkyl, alkyl, alkenyl, alkynyl or alkoxy]
or its salt, solvate, or physiologically functional derivative:
excluding the following compounds:
3-methyl-2-nitro-5-(piperidin-1-yl)thiophene;
3-ethyl-2-nitro-5-(piperidin-1-yl)thiophene;
2-nitro-5-(piperidin-1-yl)-3-(n-propyl)thiophene;
2-nitro-5-(piperidin-1-yl)-3-(i-propyl)thiophene;
3- (t-butyl)-2-nitro-5-(piperidin-1-yl)thiophene; and
3-(n-hexyl)-2-nitro-5-(piperidin-1-yl)thiophene.

2. The compound according to claim 1, wherein the alkyl is a C1-C6 alkyl, the alkenyl is a C2-C6 alkenyl, the alkynyl is a C2-C6 alkynyl, the haloalkyl is a C1-C6 haloalkyl, the cycloalkyl is a C3-C6 cycloalkyl, the alkylene is a C1-C6 alkylene and the alkoxy is a C1-C6 alkoxy.

3. The compound according to claim 2, wherein the alkylene is a C1-C2 alkylene, the haloalkyl is trifluoromethyl and the cycloalkyl is cyclopropyl.

4. The compound according to any one of claims 1 to 3, wherein R² represents cyano, formyl, halogen or haloalkyl.

5. The compound according to claim 4, wherein R² is cyano.

6. The compound according to any one of claims 1 to 5, wherein R³ is cyano, halogen or haloalkyl.

7. The compound according to claim 6, wherein R³ is a halogen or haloalkyl.

8. The compound according to claim 7, wherein R³ is bromo or trifluoromethyl.

9. The compound according to any one of claims 1 to 8, wherein Q¹ is methylene or ethylene.

10. The compound according to any one of claims 1 to 9, wherein R⁶ is hydrogen, alkyl, cycloalkyl, alkenyl, haloalkyl or alkoxy.

11. The compound according to any one of claims 1 to 10, wherein R⁶ is cyclopropyl, vinyl, trifluoromethyl or methoxy.

12. The compound according to any one of claims 1 to 11, wherein Q² is methylene or ethylene.

13. The compound according to any one of claims 1 to 12, wherein Q³ is -C(O)- or -C(S)-.

14. The compound according to any one of claims 1 to 13, wherein R⁷ is hydrogen, alkyl, alkenyl, hydroxy, alkoxy, cyano, -N(R⁸)(R⁹) or -CH(R¹⁰)(R¹¹) in which R⁸ and R⁹ represent each independently hydrogen or alkyl, R¹⁰ and R¹¹ represent each independently alkyl, haloalkyl or hydroxy, alternatively R¹⁰ and R¹¹ together form alkylenedioxy.

15. The compound according to any one of claims 1 to 14, wherein Q⁴ is methylene optionally substituted with a substituent selected from the group consisting of a halogen, hydroxy, C1-C6 alkyl, C2-C6 alkenyl, C2-C6 alkynyl, C1-C6 haloalkyl, C3-C6 cycloalkyl and C1-C6 alkoxy.

16. The compound according to any one of claims 1 to 15, wherein Q⁵ is methylene.

17. The compound according to any one of claims 1 to 16, wherein R¹⁶ is hydrogen, cycloalkyl, alkenyl, halogen, haloalkyl, hydroxy, formyl, -CH=N-R¹⁷ or -CH(R²⁰)(R²¹) in which R¹⁷ represents hydroxy and R²⁰ and R²¹ represent each independently alkyl, haloalkyl or hydroxy (excluding a case in which R²⁰ and R²¹ are simultaneously hydroxy).

18. The compound according to claim 17, wherein either R²⁰ or R²¹ is a haloalkyl and another is hydroxy.

19. The compound according to claim 18, wherein R²⁰ or R²¹ is trifluoromethyl.

20. A compound selected from among
3-bromo-5-[(cyclopropylmethyl)(propyl)amino]thiophene-2-carbaldehyde;
3-bromo-5-[(cyclopropylmethyl)(propyl)amino]thiophene-2-carbonitrile;
3-chloro-5-[(cyclopropylmethyl)(propyl)amino]thiophene-2-carbonitrile;
5-[(cyclopropylmethyl)(propyl)amino]-2-(trifluoromethyl)thiophene-2-carbaldehyde;
5-[(cyclopropylmethyl)(propyl)amino]-3-(trifluoromethyl)thiophene-2-carbonitrile;
2-bromo-5-[(cyclopropylmethyl)(propyl)amino]thiophene-3-carbonitrile;
5-[(cyclopropylmethyl)(propyl)amino]-2-(trifluoromethyl)thiophene-3-carbonitrile;
3-bromo-5-(pyrrolidin-1-yl)thiophene-2-carbonitrile;
3-bromo-5-(2-methylpiperidin-1-yl)thiophene-2-carbonitrile;
3-bromo-5-(piperidin-1-yl)thiophene-2-carbonitrile;
3-bromo-5-(3,5-dimethylpiperidin-1-yl)thiophene-2-carbonitrile;
3-bromo-5-(4-methylpiperidin-1-yl)thiophene-2-carbonitrile;
3-bromo-5-[(methyl)(2-methylpropyl)amino]thiophene-2-carbonitrile;
3-bromo-5- (dipropylamino)thiophene-2-carbonitrile;
3-bromo-5- (diallylamino)thiophene-2-carbonitrile;
5-[bis(2-methoxyethyl)amino]-3-bromothiophene-2-carbonitrile;
3-bromo-5-(2-ethylpiperidin-1-yl)thiophene-2-carbonitrile;
3-bromo-5-(2-propylpiperidin-1-yl)thiophene-2-carbonitrile;
N-(4-bromo-5-cyanothiophen-2-yl)-N-propylacetamide;
N-(4-bromo-5-cyanothiophen-2-yl)-N-propylpropanamide;
N-(4-bromo-5-cyanothiophen-2-yl)-N-propylbutanamide;
N-(4-bromo-5-cyanothiophen-2-yl)-2-methyl-N-propylpropanamide;
3-bromo-5-[(propyl)(2,2,2-trifluoroethyl)amino]thiophene-2-carbonitrile;
N-(4-bromo-5-cyanothiophen-2-yl)-N-propylglycine ethyl ester;
N2-(4-bromo-5-cyanothiophen-2-yl)-N-mothyl-N2-propylglycinamide;
N-(4-bromo-5-cyanothiophen-2-yl)-N-propylglycine;
N2-(4-bromo-5-cyanothiophen-2-yl)-N2-propylglycinamide;
3-bromo-5-[(cyanomethyl)(propyl)amino]thiophene-2-carbonitrile;
N2-(4-bromo-5-cyanothiophen-2-yl)-N,N-dimethyl-N2-propylglycinamide;
3-bromo-5-[(1,3-dioxolan-2-ylmethyl)amino]thiophene-2-carbonitrile;
3-bromo-5-[(1,3-dioxolan-2-ylmethyl)(propyl)amino]thiophene-2-carbonitrile;
3-bromo-5-[(2-oxoethyl)(propyl)amino]thiophene-2-carbonitrile;
3-bromo-5-[propyl(3,3,3-trifluoro-2-hydroxypropyl)amino]thiophene-2-carbonitrile;
3-bromo-5-[(2-hydroxypropyl)(propyl)amino]thiophene-2-carbonitrile;
3-bromo-5-(2-ethyl-6-oxopiperidin-1-yl)thiophene-2-carbonitrile;
3-bromo-5-(2-ethyl-5-thioxopiperidin-1-yl)thiophene-2-carbonitrile;
N-(4-bromo-5-cyanothiophen-2-yl)-N-propylpropanthioamide; 3-bromo-5-[2-(hydroxymethyl)piperidin-1-yl]thiophene-2-carbonitrile;
3-bromo-5-(2-formylpiperidin-1-yl)thiophene-2-carbonitrile;
3-bromo-5-[2-(2,2,2-trifluoro-1-hydroxyethyl)piperidin-1-y1]thiophene-2-carbonitrile;
3-bromo-5-[2-(1-hydroxyethyl)piperidin-1-yl]thiophene-2-carbonitrile;
3-bromo-5-{[2-(hydroxyimino)ethyl](propyl)amino}thiophene-2-carbonitrile;
5-[2-(2,2,2-trifluoro-1-hydroxyethyl)piperidin-1-yl]-3-(trifluoromethyl)thiophene-2-carbonitrile;
5-[(propyl)(2,2,2-trifluoroethyl)amino]-3-trifluoromethylthiophene-2-carbonitrile;
5-[(propyl)(2,2,2-trifluoroethyl)amino]-3-methylthiophene-2-carbonitrile or
5-(2-cyanopiperidin-1-yl)-3-bromothiophene-2-carbonitrile,
or its salt, solvate or physiologically functional derivative.

21. The compound according to any one of claims 1 to 20, for use as an active therapeutic substance.

22. The compound according to any one of claims 1 to 20, for use in treatment or prevention of conditions or disorders having sensitivity to selective androgen receptor modulation.

23. The compound according to claim 22, wherein the condition or disorder having sensitivity to selective androgen receptor modulation is an androgen-dependent disease, proliferative disease or amyloidosis.

24. The compound according to claim 22, wherein the condition or disorder having sensitivity to selective androgen receptor modulation is a malignant tumor containing an androgen receptor of breast, brain, skin, ovary, bladder, lymph node, liver, kidney, uterus, spleen, endometrium, lung, colon or prostate; prostatic hypertrophy, ADAM, trichosis, acne, seborrhea, androgenic alopecia, excessive hair growth, libido regression, sexual dysfunction, muscular weakness, aorta smooth muscle cell proliferation, Alzheimer's disease, mad cow disease, new variant Creutzfeldt-Jacob disease, familial amyloid polyneuropathy, osteoporosis, dyslipidemia, obesity, diabetes mellitus, depression, urine incontinence, arterial sclerosis, uterine fibroid disease or endometriosis.

25. A pharmaceutical composition comprising the compound according to any one of claims 1 to 20 and a pharmaceutically acceptable carrier.

26. Use of the compound according to any one of claims 1 to 20 in production of a medicine for use in treatment or prevention of conditions or disorders having sensitivity to selective androgen receptor modulation.

27. The use according to claim 26, wherein the condition or disorder having sensitivity to selective androgen receptor modulation is an androgen-dependent disease, proliferative disease or amyloidosis.

28. A method of treatment or prevention of conditions or disorders having sensitivity to selective androgen receptor modulation, comprising a step of administering a therapeutically effective amount of the compound according to any one of claims 1 to 20 to a subject in need of the treatment or prevention.

29. The method according to claim 28, wherein the condition or disorder having sensitivity to selective androgen receptor modulation is an androgen-dependent disease, proliferative disease or amyloidosis.

30. The method according to claim 28, wherein the condition or disorder having sensitivity to selective androgen receptor modulation is a malignant tumor containing an androgen receptor of breast, brain, skin, ovary, bladder, lymph node, liver, kidney, uterus, spleen, endometrium, lung, colon or prostate; prostatic hypertrophy, ADAM, trichosis, acne, seborrhea, androgenic alopecia, excessive hair growth, libido regression, sexual dysfunction, muscular weakness, aorta smooth muscle cell proliferation, Alzheimer's disease, mad cow disease, new variant Creutzfeldt-Jacob disease, familial amyloid polyneuropathy, osteoporosis, dyslipidemia, obesity, diabetes mellitus, depression, urine incontinence, arterial sclerosis, uterine fibroid disease or endometriosis.


SEQUENCE LISTING

<110> SUMITOMO CHEMICAL COMPANY, LIMITED

<120> Thiophene derivatives

<130> S23494WO01

<150> JP 2009-110804
<151> 2009-04-30

<160> 9

<210> 1
<211> 35
<212> DNA
<213> Artificial Sequence

<220>
<223> Designed oligonucleotide primer for PCR

<400> 1
gaggcggggt aagggaagta ggtggaagat tcagc       35

<210> 2
<211> 35
<212> DNA
<213> Artificial Sequence

<220>
<223> Designed oligonucleotide primer for PCR

<400> 2
gggtggggaa atagggtttc caatgcttca ctggg       35

<210> 3
<211> 918
<212> PRT
<213> Homo sapiens

<400> 3
Met Glu Val Gln Leu Gly Leu Gly Arg Val Tyr Pro Arg Pro Pro Ser
1               5                   10                  15
Lys Thr Tyr Arg Gly Ala Phe Gln Asn Leu Phe Gln Ser Val Arg Glu
            20                  25                  30
Val Ile Gln Asn Pro Gly Pro Arg His Pro Glu Ala Ala Ser Ala Ala
        35                  40                  45
Pro Pro Gly Ala Ser Leu Leu Leu Leu Gln Gln Gln Gln Gln Gln Gln
    50                  55                  60
Gln Gln Gln Gln Gln Gln Gln Gln Gln Gln Glu Thr Ser Pro Arg Gln
65                  70                  75                  80
Gln Gln Gln Gln Gln Gly Glu Asp Gly Ser Pro Gln Ala His Arg Arg
                85                  90                  95
Gly Pro Thr Gly Tyr Leu Val Leu Asp Glu Glu Gln Gln Pro Ser Gln
            100                 105                 110
Pro Gln Ser Ala Leu Glu Cys His Pro Glu Arg Gly Cys Val Pro Glu
        115                 120                 125
Pro Gly Ala Ala Val Ala Ala Ser Lys Gly Leu Pro Gln Gln Leu Pro
    130                 135                 140
Ala Pro Pro Asp Glu Asp Asp Ser Ala Ala Pro Ser Thr Leu Ser Leu
145                 150                 155                 160
Leu Gly Pro Thr Phe Pro Gly Leu Ser Ser Cys Ser Ala Asp Leu Lys
                165                 170                 175
Asp Ile Leu Ser Glu Ala Ser Thr Met Gln Leu Leu Gln Gln Gln
            180                 185                 190

Gln Glu Ala Val Ser Glu Gly Ser Ser Ser Gly Arg Ala Arg Glu Ala
        195                 200                 205
Ser Gly Ala Pro Thr Ser Ser Lys Asp Asn Tyr Leu Gly Gly Thr Ser
    210                 215                 220
Thr Ile Ser Asp Asn Ala Lys Glu Leu Cys Lys Ala Val Ser Val Ser
225                 230                 235                 240
Met Gly Leu Gly Val Glu Ala Leu Glu His Leu Ser Pro Gly Glu Gln
                245                 250                 255
Leu Arg Gly Asp Cys Met Tyr Ala Pro Leu Leu Gly Val Pro Pro Ala
            260                 265                 270
Val Arg Pro Thr Pro Cys Ala Pro Leu Ala Glu Cys Lys Gly Ser Leu
        275                 280                 285
Leu Asp Asp Ser Ala Gly Lys Ser Thr Glu Asp Thr Ala Glu Tyr Ser
    290                 295                 300
Pro Phe Lys Gly Gly Tyr Thr Lys Gly Leu Glu Gly Glu Ser Leu Gly
305                 310                 315                 320
Cys Ser Gly Ser Ala Ala Ala Gly Ser Ser Gly Thr Leu Glu Leu Pro
                325                 330                 335
Ser Thr Leu Ser Leu Tyr Lys Ser Gly Ala Leu Asp Glu Ala Ala Ala
            340                 345                 350
Tyr Gln Ser Arg Asp Tyr Tyr Asn Phe Pro Leu Ala Leu Ala Gly Pro
        355                 360                 365
Pro Pro Pro Pro Pro Pro Pro His Pro His Ala Arg Ile Lys Leu Glu
    370                 375                 380
Asn Pro Leu Asp Tyr Gly Ser Ala Trp Ala Ala Ala Ala Ala Gln Cys
385                 390                 395                 400
Arg Tyr Gly Asp Leu Ala Ser Leu His Gly Ala Gly Ala Ala Gly Pro
                405                 410                 415
Gly Ser Gly Ser Pro Ser Ala Ala Ala Ser Ser Ser Trp His Thr Leu
            420                 425                 430
Phe Thr Ala Glu Glu Gly Gln Leu Tyr Gly Pro Cys Gly Gly Gly Gly
        435                 440                 445
Gly Gly Gly Gly Gly Gly Gly Gly Gly Gly Gly Gly Gly Gly Gly Gly
    450                 455                 460
Gly Gly Gly Gly Gly Gly Gly Glu Ala Gly Ala Val Ala Pro Tyr Gly
465                 470                 475                 480
Tyr Thr Arg Pro Pro Gln Gly Leu Ala Gly Gln Glu Ser Asp Phe Thr
                485                 490                 495
Ala Pro Asp Val Trp Tyr Pro Gly Gly Met Val Ser Arg Val Pro Tyr
            500                 505                 510
Pro Ser Pro Thr Cys Val Lys Ser Glu Met Gly Pro Trp Met Asp Ser
        515                 520                 525
Tyr Ser Gly Pro Tyr Gly Asp Met Arg Leu Glu Thr Ala Arg Asp His
    530                 535                 540
Val Leu Pro Ile Asp Tyr Tyr Phe Pro Pro Gln Lys Thr Cys Leu Ile
545                 550                 555                 560
Cys Gly Asp Glu Ala Ser Gly Cys His Tyr Gly Ala Leu Thr Cys Gly
                565                 570                 575
Ser Cys Lys Val Phe Phe Lys Arg Ala Ala Glu Gly Lys Gln Lys Tyr
            580                 585                 590
Leu Cys Ala Ser Arg Asn Asp Cys Thr Ile Asp Lys Phe Arg Arg Lys
        595                 600                 605
Asn Cys Pro Ser Cys Arg Leu Arg Lys Cys Tyr Glu Ala Gly Met Thr
    610                 615                 620
Leu Gly Ala Arg Lys Leu Lys Lys Leu Gly Asn Leu Lys Leu Gln Glu
625                 630                 635                 640
Glu Gly Glu Ala Ser Ser Thr Thr Ser Pro Thr Glu Glu Thr Thr Gln
                645                 650                 655
Lys Leu Thr Val Ser His Ile Glu Gly Tyr Glu Cys Gln Pro Ile Phe
            660                 665                 670
Leu Asn Val Leu Glu Ala Ile Glu Pro Gly Val Val Cys Ala Gly His
        675                 680                 685
Asp Asn Asn Gln Pro Asp Ser Phe Ala Ala Leu Leu Ser Ser Leu Asn
    690                 695                 700
Glu Leu Gly Glu Arg Gln Leu Val His Val Val Lys Trp Ala Lys Ala

705                 710                 715                 720
Leu Pro Gly Phe Arg Asn Leu His Val Asp Asp Gln Met Ala Val Ile
                725                 730                 735
Gln Tyr Ser Trp Met Gly Leu Met Val Phe Ala Met Gly Trp Arg Ser
            740                 745                 750
Phe Thr Asn Val Asn Ser Arg Met Leu Tyr Phe Ala Pro Asp Leu Val
        755                 760                 765
Phe Asn Glu Tyr Arg Met His Lys Ser Arg Met Tyr Ser Gln Cys Val
    770                 775                 780
Arg Met Arg His Leu Ser Gln Glu Phe Gly Trp Leu Gln Ile Thr Pro
785                 790                 795                 800
Gln Glu Phe Leu Cys Met Lys Ala Leu Leu Leu Phe Ser Ile Ile Pro
                805                 810                 815
Val Asp Gly Leu Lys Asn Gln Lys Phe Phe Asp Glu Leu Arg Met Asn
            820                 825                 830
Tyr Ile Lys Glu Leu Asp Arg Ile Ile Ala Cys Lys Arg Lys Asn Pro
        835                 840                 845
Thr Ser Cys Ser Arg Arg Phe Tyr Gln Leu Thr Lys Leu Leu Asp Ser
    850                 855                 860
Val Gln Pro Ile Ala Arg Glu Leu His Gln Phe Thr Phe Asp Leu Leu
865                 870                 875                 880
Ile Lys Ser His Met Val Ser Val Asp Phe Pro Glu Met Met Ala Glu
                885                 890                 895
Ile Ile Ser Val Gln Val Pro Lys Ile Leu Ser Gly Lys Val Lys Pro
            900                 905                 910
Ile Tyr Phe His Thr Gln
        915         918

<210> 4
<211> 40
<212> DNA
<213> Artificial Sequence

<220>
<223> Designed oligonucleotide primer for PCR

<400> 4
cccagccacc atggaagtgc agttagggct gggaagggtc      40

<210> 5
<211> 35
<212> DNA
<213> Artificial Sequence

<220>
<223> Designed oligonucleotide primer for PCR

<400> 5
gggtggggaa atagggtttc caatgcttca ctggg           35

<210> 6
<211> 32
<212> DNA
<213> Artificial Sequence

<220>
<223> Designed oligonucleotide

<400> 6
ctagtaaagt actccaagaa cctatttgag ct              32

<210> 7
<211> 32
<212> DNA
<213> Artificial Sequence

<220>
<223> Designed oligonucleotide

<400> 7
caaataggtt cttggagtac tttactagag ct          32

<210> 8
<211> 52
<212> DNA
<213> Artificial Sequence

<220>
<223> Designed oligonucleotide

<400> 8
gatctcgact ataaagaggg caggctgtcc tctaagcgtc accacgactt ca          52

<210> 9
<211> 52
<212> DNA
<213> Artificial Sequence

<220>
<223> Designed oligonucleotide

<400> 9
agcttgaagt cgtggtgacg cttagaggac agcctgccct ctttatagtc ga          52